# EUROPEAN PATENT SPECIFICATION

(11) **EP 4 433 467 B1**
(45) Date of publication and mention of the grant of the patent: **31.12.2025**
(21) Application number: 22818305.9
(22) Date of filing: 15.11.2022
(51) Int. Cl.: C07D 401/04, C07D 403/04, C07D 409/04, C07D 491/056, A01N 43/58

(54) **HERBICIDAL DERIVATIVES**
HERBIZIDE DERIVATE
DÉRIVÉS HERBICIDES

(30) Priority: 18.11.2021 EP 21208993
(43) Date of publication of application: 25.09.2024
(73) Proprietor: Syngenta Crop Protection AG, 4058 Basel (CH)
(72) Inventor: MORRIS, James Alan, Bracknell Berkshire RG42 6EY (US); WHALLEY, Louisa, Bracknell Berkshire RG42 6EY (US); ANDERSON, Zoe Jane, Bracknell Berkshire RG42 6EY (US)
(74) Representative: SYNGENTA IP
(86) International application number: PCT/EP2022/082045
(87) International publication number: WO 2023/088921

(56) References cited:
- EP-A1- 0 363 236
- EP-A2- 0 320 793
- WO-A1-2021/233786
- US-A- 5 129 939

## Description

The present invention relates to herbicidal cinnoline derivatives, e.g., as active ingredients, which have herbicidal activity. The invention also relates to agrochemical compositions which comprise at least one of the cinnoline derivatives, to processes of preparation of these compounds and to uses of the cinnoline derivatives or compositions in agriculture or horticulture for controlling weeds, in particular in crops of useful plants.

EP0273325, EP0274717, EP0320793, US5183891, WO2021/233786, and WO2021/233787 describe cinnoline derivatives as herbicidal agents.

According to the present invention, there is provided a compound of Formula (I): 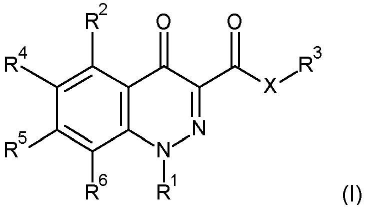 wherein
X is O, NR⁷ or S;
R¹ is heteroaryl, wherein the heteroaryl moiety is a 5- or 6-membered aromatic ring which comprises 1, 2, or 3 heteroatoms individually selected from N, O and S, and wherein the heteroaryl moiety is optionally substituted with 1, 2, 3, or 4 groups, which may be the same or different, represented by R⁸;
R² is halogen, cyano, cyanoC₁-C₆alkyl, C₁-C₆haloalkyl, C₁-C₆alkylcarbonyl, C₂-C₆alkenyl, C₂-C₆alkynyl, C₁-C₆alkoxyC₂-C₆alkenyl, C₂-C₆alkenyloxyC₁-C₆alkyl, -CR¹¹=N-OR¹⁰, nitro, S(O)ₙC₁-C₆alkyl, S(O)ₙC₁-C₆haloalkyl, or S(O)ₙC₃-C₆cycloalkyl;
R³ is hydrogen, C₁-C₆alkyl, C₁-C₆haloalkyl, C₃-C₆cycloalkyl, C₃-C₆cycloalkylC₁-C₆alkyl, C₁-C₆alkoxyC₁-C₆alkyl, C₂-C₆alkenyl, C₂-C₆alkynyl, phenyl, or phenylC₁-C₃alkyl, wherein the phenyl moieties may be optionally substituted with 1, 2, 3, or 4 groups, which may be the same or different, represented by R¹²;
R⁴, R⁵, and R⁶ are each independently selected from hydrogen, halogen, cyano, C₁-C₆alkyl, C₁-C₆alkoxy, C₁-C₆haloalkyl, C₁-C₆haloalkoxy, C₁-C₆alkylsulfanyl, C₁-C₆alkylsulfinyl, and C₁-C₆alkylsulfonyl;
R⁷ is hydrogen, C₁-C₃alkyl, or C₁-C₃alkoxy;
R⁸ is halogen, cyano, C₁-C₆alkyl, C₁-C₆alkoxy, C₁-C₆haloalkyl, C₁-C₆haloalkoxy, C₁-C₆alkylsulfanyl, or C₁-C₆alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the aryl, heterocyclyl, or heteroaryl rings may be optionally substituted with 1, 2, 3, or 4 groups, which may be the same or different, represented by R⁹;
n is 0, 1 or 2;
R⁹ is halogen, C₁-C₃alkyl, C₁-C₃haloalkyl, or C₁-C₃alkoxy;
R¹⁰ and R¹¹ are each independently selected from hydrogen and C₁-C₃alkyl;
R¹² is halogen, cyano, C₁-C₃alkyl, or C₁-C₃alkoxy;
or a salt or an N-oxide thereof.

Surprisingly, it has been found that the novel compounds of Formula (I) have, for practical purposes, a very advantageous level of herbicidal activity.

According to a second aspect of the invention, there is provided an agrochemical composition comprising a herbicidally effective amount of a compound of Formula (I) according to the present invention. Such an agricultural composition may further comprise at least one additional active ingredient and/or an agrochemically-acceptable diluent or carrier.

According to a third aspect of the invention, there is provided a method of controlling weeds at a locus comprising applying to the locus a weed controlling amount of a composition comprising a compound of Formula (I).

According to a fourth aspect of the invention, there is provided the use of a compound of Formula (I) as a herbicide.

Where substituents are indicated as being "optionally substituted", this means that they may or may not carry one or more identical or different substituents, e.g., one, two or three R⁸ substituents. For example, C₁-C₈alkyl substituted by 1, 2 or 3 halogens, may include, but not be limited to, -CH₂Cl, -CHCl₂, -CCl₃, -CH₂F, -CHF₂, -CF₃, -CH₂CF₃ or -CF₂CH₃ groups. As another example, C₁-C₆alkoxy substituted by 1, 2 or 3 halogens, may include, but not limited to, CH₂ClO-, CHCl₂O-, CCl₃O-, CH₂FO-, CHF₂O-, CF₃O-, CF₃CH₂O- or CH₃CF₂O- groups.

As used herein, the terms "cyano" and "nitrile" mean a -CN group.

As used herein, the term "halogen" refers to fluorine (fluoro), chlorine (chloro), bromine (bromo) or iodine (iodo).

As used herein, the term "hydroxy" means an -OH group.

As used herein, the term "acetyl" means a -C(O)CH₃ group.

As used herein, the term "nitro" means an NO₂ group.

As used herein, the term "C₁-C₆alkyl" refers to a straight or branched hydrocarbon chain radical consisting solely of carbon and hydrogen atoms, containing no unsaturation, having from one to six carbon atoms, and which is attached to the rest of the molecule by a single bond. "C₁-C₄alkyl" and "C₁-C₃alkyl" are to be construed accordingly. Examples of C₁-C₆alkyl include, but are not limited to, methyl, ethyl, n-propyl, and the isomers thereof, for example, iso-propyl. A "C₁-C₆alkylene" group refers to the corresponding definition of C₁-C₆alkyl, except that such radical is attached to the rest of the molecule by two single bonds. The term "C₁-C₂alkylene" is to be construed accordingly. Examples of C₁-C₆alkylene, include, but are not limited to, -CH₂-, -CH₂CH₂- and -(CH₂)₃-.

As used herein, the term "C₁-C₆haloalkyl" refers a C₁-C₆alkyl radical as generally defined above substituted by one or more of the same or different halogen atoms. The terms "C₁-C₄haloalkyl" and "C₁-C₃haloalkyl", are to be construed accordingly. Examples of C₁-C₆haloalkyl include, but are not limited to difluoromethyl, trifluoromethyl, and 2,2,2-trifluoromethyl.

As used herein, the term "cyanoC₁-C₆alkyl" refers to a C₁-C₆alkyl radical as generally defined above substituted by one or more cyano groups, as defined above. Examples of cyanoC₁-C₆alkyl include, but are not limited to 2-cyanomethyl and 2-cyanoethyl.

As used herein, the term "C₁-C₆haloalkoxy" refers to a C₁-C₆alkoxy radical as generally defined above substituted by one or more of the same or different halogen atoms. The terms "C₁-C₄haloalkoxy" and "C₁-C₃haloalkoxy", are to be construed accordingly. Examples of C₁-C₆haloalkoxy include, but are not limited to trifluoromethoxy.

As used herein, the term "C₁-C₆alkoxy" refers to a radical of the formula -ORₐ where Rₐ is a C₁-C₆alkyl radical as generally defined above. The terms "C₁-C₄alkoxy" and "C₁-C₃alkoxy" are to be construed accordingly. Examples of C₁-C₆alkoxy include, but are not limited to, methoxy, ethoxy, 1-methylethoxy (iso-propoxy), and propoxy.

As used herein, the term "C₂-C₆alkenyl" refers to a straight or branched hydrocarbon chain radical group consisting solely of carbon and hydrogen atoms, containing at least one double bond that can be of either the (E)- or (Z)-configuration, having from two to six carbon atoms, which is attached to the rest of the molecule by a single bond. The term "C₂-C₃alkenyl" is to be construed accordingly. Examples of C₂-C₆alkenyl include, but are not limited to, ethenyl (vinyl), prop-1-enyl, prop-2-enyl (allyl), and but-1-enyl.

As used herein, the term "C₂-C₆alkynyl" refers to a straight or branched hydrocarbon chain radical group consisting solely of carbon and hydrogen atoms, containing at least one triple bond, having from two to six carbon atoms, and which is attached to the rest of the molecule by a single bond. The term "C₂-C₃alkynyl" is to be construed accordingly. Examples of C₂-C₆alkynyl include, but are not limited to, ethynyl, prop-1-ynyl, and but-1-ynyl.

As used herein, the term "C₁-C₆alkoxyC₁-C₆alkyl" refers to a radical of the formula R_{b}ORₐ- wherein R_{b} is a C₁-C₆alkyl radical as generally defined above, and Rₐ is a C₁-C₆alkylene radical as generally defined above. Examples of C₁-C₆alkoxyC₁-C₆alkyl include, but are not limited to, methoxymethyl, methoxyethyl, and ethoxymethyl.

As used herein, the term "C₃-C₆cycloalkyl" refers to a radical which is a monocyclic saturated ring system which contains 3 to 6 carbon atoms. The terms "C₃-C₅cycloalkyl" and "C₃-C₄cycloalkyl" are to be construed accordingly. Examples of C₃-C₆cycloalkyl include, but are not limited to, cyclopropyl, cyclobutyl, cyclopentyl and cyclohexyl.

As used herein, the term "C₃-C₆cycloalkylC₁-C₆alkyl" refers to a C₃-C₆cycloalkyl ring attached to the rest of the molecule by a C₁-C₆alkylene linker as defined above. Examples of C₃-C₆cycloalkylC₁-C₆alkyl include, but are not limited to, cyclopropylmethyl, cyclobutylmethyl, cyclopentlymethyl, and cyclohexylmethyl.

As used herein, the term "C₁-C₆alkoxyC₂-C₆alkenyl" refers to a radical of the formula R_{b}ORₐ-wherein R_{b} is a C₁-C₆alkyl radical as generally defined above, and Rₐ is a C₁-C₆alkene radical as generally defined above. Examples of C₁-C₆alkoxyC₂-C₆alkenyl include, but are not limited to 1-methoxyvinyl and 1-ethoxyvinyl.

As used herein, the term "C₂-C₆alkenyloxyC₁-C₆alkyl" refers to a radical of the formula R_{b}ORₐ-wherein R_{b} is a C₂-C₆alkenyl radical as generally defined above, and Rₐ is a C₁-C₆alkylene radical as generally defined above.

As used herein, the term "phenoxy" refers to a phenyl ring attached to the rest of the molecule through an oxygen atom.

As used herein, the term "phenylC₁-C₃alkyl" refers to a phenyl ring attached to the rest of the molecule by a C₁-C₃alkylene linker as defined above.

As used herein, the term "heteroaryl" refers to a 5- or 6-membered aromatic monocyclic ring radical which comprises 1, 2, 3, or 4 heteroatoms individually selected from N, O and S. The heteroaryl radical may be bonded to the rest of the molecule *via* a carbon atom or heteroatom. Examples of heteroaryl include, but are not limited to, furanyl, benzofuranyl, thiophenyl, benzothiophenyl, benzothiazolyl, imidazolyl, benzimidazolyl, oxadiazolyl, pyridyl, pyrrolyl, quinolinyl, isoquinolinyl, indolyl, isoindolyl, indazolyl, pyrazolyl, thiazolyl, oxazolyl, benzoxazolyl, pyridazinyl, cinnolinyl, pyrimidinyl, and quinazolinyl.

As used herein, the term "C₁-C₆alkylcarbonyl" refers to a radical of the formula -C(O)Rₐ, where Rₐ is a C₁-C₆alkyl radical as generally defined above.

As used herein, the term "C₁-C₆alkylsulfanyl" refers to a radical of the formula -SRₐ, where Rₐ is a C₁-C₆alkyl radical as generally defined above. The terms "C₁-C₄alkylsulfanyl" and "C₁-C₃alkylsulfanyl", are to be construed accordingly. Examples of C₁-C₆alkylsulfanyl include, but are not limited to methylsulfanyl.

As used herein, the term "C₁-C₆alkylsulfinyl" refers to a radical of the formula -S(O)Rₐ, where Rₐ is a C₁-C₆alkyl radical as generally defined above. The terms "C₁-C₄alkylsulfinyl" and "C₁-C₃alkylsulfinyl", are to be construed accordingly. Examples of C₁-C₆alkylsulfinyl include, but are not limited to methylsulfinyl.

As used herein, the term "C₁-C₆alkylsulfonyl" refers to a radical of the formula -S(O)₂Rₐ, where Rₐ is a C₁-C₆alkyl radical as generally defined above. The terms "C₁-C₄alkylsulfonyl" and "C₁-C₃alkylsulfonyl", are to be construed accordingly. Examples of C₁-C₆alkylsolfanyl include, but are not limited to methylsulfonyl.

The presence of one or more possible stereogenic elements in a compound of Formula (I) means that the compounds may occur in optically isomeric forms, i.e., enantiomeric or diastereomeric forms. Also, atropisomers may occur as a result of restricted rotation about a single bond. Formula (I) is intended to include all those possible isomeric forms and mixtures thereof. The present invention includes all those possible isomeric forms and mixtures thereof for a compound of Formula (I). Likewise, Formula (I) is intended to include all possible tautomers. The present invention includes all possible tautomeric forms for a compound of Formula (I). In each case, the compounds of Formula (I) according to the invention are in free form, in oxidized form as an N-oxide, or in salt form, e.g., an agronomically usable salt form. Salts that the compounds of Formula (I) may form with amines, including primary, secondary and tertiary amines (for example ammonia, dimethylamine and triethylamine), alkali metal and alkaline earth metal bases, transition metals or quaternary ammonium bases are preferred. N-oxides are oxidized forms of tertiary amines or oxidized forms of nitrogen-containing heteroaromatic compounds. They are described for instance in the book "Heterocyclic N-oxides" by A. Albini and S. Pietra, CRC Press, Boca Raton (1991).

The following list provides definitions, including preferred definitions, for substituents X, R¹, R², R³, R⁴, R⁵, R⁶, R⁷, n, R⁸, R⁹, R¹⁰, R¹¹, and R¹², with reference to compounds of Formula (I). For any one of these substituents, any of the definitions given below may be combined with any definition of any other substituent given below or elsewhere in this document.

X is O, NR⁷ or S. In one set of embodiments, X is O. In another set of embodiments, X is S. In a further set of embodiments, X is NR⁷.

R¹ is heteroaryl, wherein the heteroaryl moiety is a 5- or 6-membered aromatic ring which comprises 1, 2, or 3 heteroatoms individually selected from N, O and S, and wherein the heteroaryl moiety is optionally substituted with 1, 2, 3, or 4 groups, which may be the same or different, represented by R⁸. Preferably, R¹ is heteroaryl, wherein the heteroaryl moiety is a 5- or 6-membered aromatic ring which comprises 1, 2, or 3 heteroatoms individually selected from N, O and S, and wherein the heteroaryl moiety is optionally substituted with 1, 2, or 3 groups, which may be the same or different, represented by R⁸. More preferably, R¹ is heteroaryl, wherein the heteroaryl moiety is a 5- or 6-membered aromatic ring which comprises 1, 2, or 3 heteroatoms individually selected from N, O and S, and wherein the heteroaryl moiety is optionally substituted with 1 or 2 groups, which may be the same or different, represented by R⁸. Even more preferably, R¹ is heteroaryl, wherein the heteroaryl moiety is a 5- or 6-membered aromatic ring which comprises 1 or 2 heteroatoms individually selected from N and S, and wherein the heteroaryl moiety is optionally substituted with 1 or 2 groups, which may be the same or different, represented by R⁸. More preferably still, R¹ is heteroaryl, wherein the heteroaryl moiety is a 6-membered aromatic ring which comprises 1 or 2 nitrogen heteroatoms, and wherein the heteroaryl moiety is optionally substituted with 1 or 2 groups, which may be the same or different, represented by R⁸.

In one embodiment, R¹ is pyridyl or pyrimidinyl, wherein each pyridyl or pyrimidinyl moiety is optionally substituted with 1 or 2 groups, which may be the same or different, represented by R⁸. In another embodiment, R¹ is pyridyl or pyrimidinyl, wherein each pyridyl is moiety is substituted with 1 or 2 groups, which may be the same or different, represented by R⁸.

R² is halogen, cyano, cyanoC₁-C₆alkyl, C₁-C₆haloalkyl, C₁-C₆alkylcarbonyl, C₂-C₆alkenyl, C₂-C₆alkynyl, C₁-C₆alkoxyC₂-C₆alkenyl, C₂-C₆alkenyloxyC₁-C₆alkyl, -CR¹¹=N-OR¹⁰, nitro, S(O)ₙC₁-C₆alkyl, S(O)ₙC₁-C₆haloalkyl, or S(O)ₙC₃-C₆cycloalkyl. Preferably, R² is halogen, cyano, cyanoC₁-C₄alkyl, C₁-C₄haloalkyl, C₁-C₆alkylcarbonyl, C₂-C₄alkenyl, C₂-C₄alkynyl, C₁-C₆alkoxyC₂-C₆alkenyl, C₂-C₄alkenyloxyC₁-C₄alkyl, -CR¹¹=N-OR¹⁰, nitro, S(O)ₙC₁-C₆alkyl, S(O)ₙC₁-C₄haloalkyl, or S(O)ₙC₃-C₆cycloalkyl. More preferably, R² is halogen, cyano, C₁-C₆alkylcarbonyl, C₁-C₆alkoxyC₂-C₆alkenyl, - CR¹¹=N-OR¹⁰, or S(O)ₙC₁-C₆alkyl. Even more preferably, R² is halogen, cyano, C₁-C₄alkylcarbonyl, C₁-C₄alkoxyC₂-C₄alkenyl, -CR¹¹=N-OR¹⁰, or S(O)₂C₁-C₄alkyl.

In one set of embodiments, R² is halogen, cyano, acetyl, 1-methoxyvinyl, 1-ethoxyvinyl, methylsulfonyl, or N-methoxy-C-methyl-carbonimidoyl. In another set of embodiments, R² is halogen, cyano, acetyl, or N-methoxy-C-methyl-carbonimidoyl. In a further set of embodiments, R² is bromo, cyano, acetyl, or N-methoxy-C-methyl-carbonimidoyl. In a still further set of embodiments, R² is halogen or cyano, and preferably, bromo or cyano.

R³ is hydrogen, C₁-C₆alkyl, C₁-C₆haloalkyl, C₃-C₆cycloalkyl, C₃-C₆cycloalkylC₁-C₆alkyl, C₁-C₆alkoxyC₁-C₆alkyl, C₂-C₆alkenyl, C₂-C₆alkynyl, phenyl, or phenylC₁-C₃alkyl, wherein the phenyl moieties may be optionally substituted with 1, 2, 3, or 4 groups, which may be the same or different, represented by R¹². Preferably, R³ is hydrogen, C₁-C₆alkyl, C₁-C₆haloalkyl, C₃-C₆cycloalkyl, C₃-C₆cycloalkylC₁-C₃alkyl, C₁-C₄alkoxyC₁-C₃alkyl, C₂-C₆alkenyl, C₂-C₆alkynyl, phenyl, or phenylC₁-C₂alkyl, wherein the phenyl moieties may be optionally substituted with 1, 2, or 3 groups, which may be the same or different, represented by R¹². More preferably, R³ is hydrogen, C₁-C₆alkyl, C₁-C₄haloalkyl, C₃-C₆cycloalkyl, C₃-C₆cycloalkylC₁-C₃alkyl, C₁-C₄alkoxyC₁-C₃alkyl, phenyl, or benzyl, wherein the phenyl moieties may be optionally substituted with 1 or 2 groups, which may be the same or different, represented by R¹². Even more preferably, R³ is hydrogen or C₁-C₄alkyl. More preferably still, R³ is hydrogen or C₁-C₃alkyl. Particularly preferred is when R³ is hydrogen, methyl, or ethyl. In one set of embodiments, R³ is hydrogen or ethyl.

R⁴, R⁵, and R⁶ are each independently selected from hydrogen, halogen, cyano, C₁-C₆alkyl, C₁-C₆alkoxy, C₁-C₆haloalkyl, C₁-C₆haloalkoxy, C₁-C₆alkylsulfanyl, C₁-C₆alkylsulfinyl, and C₁-C₆alkylsulfonyl. Preferably, R⁴, R⁵, and R⁶ are each independently selected from hydrogen, halogen, cyano, C₁-C₄alkyl, C₁-C₃alkoxy, C₁-C₃haloalkyl, C₁-C₃haloalkoxy, C₁-C₃alkylsulfanyl, C₁-C₃alkylsulfinyl, and C₁-C₃alkylsulfonyl. More preferably, R⁴, R⁵, and R⁶ are each independently selected from hydrogen, fluoro, bromo, cyano, C₁-C₄alkyl, methoxy, ethoxy, trifluoromethyl, trifluoromethoxy, methylsulfanyl, and methylsulfonyl. Even more preferably, R⁴, R⁵, and R⁶ are each independently selected from hydrogen, fluoro, bromo, cyano, methyl, isobutyl, methoxy, and trifluoromethyl. More preferably still, R⁴, R⁵, and R⁶ are each independently selected from hydrogen, fluoro, bromo, cyano, methyl, isobutyl, methoxy, and trifluoromethyl. In a preferred set of embodiments, R⁴, R⁵, and R⁶ are all hydrogen.

R⁷ is hydrogen, C₁-C₃alkyl, or C₁-C₃alkoxy. Preferably, R⁷ is hydrogen, methyl, or methoxy. More preferably, R⁷ is hydrogen.

R⁸ is halogen, cyano, C₁-C₆alkyl, C₁-C₆alkoxy, C₁-C₆haloalkyl, C₁-C₆haloalkoxy, C₁-C₆alkylsulfanyl, or C₁-C₆alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the aryl, heterocyclyl, or heteroaryl rings may be optionally substituted with 1, 2, 3 or 4 groups, which may be the same or different, represented by R⁹.

Preferably, R⁸ is halogen, cyano, C₁-C₄alkyl, C₁-C₄alkoxy, C₁-C₃haloalkyl, C₁-C₃haloalkoxy, C₁-C₃alkylsulfanyl, or C₁-C₃alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the heterocyclyl or heteroaryl rings may be optionally substituted with 1, 2, or 3 groups, which may be the same or different, represented by R⁹.

More preferably, R⁸ is halogen, cyano, C₁-C₃alkyl, C₁-C₃alkoxy, C₁-C₃haloalkyl, C₁-C₃haloalkoxy, or C₁-C₃alkylsulfanyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 oxygen atoms, or any two adjacent R⁸ groups together with the carbon atoms to which they are attached, may form a 5- or 6-membered heteroaryl ring, comprising 1 or 2 nitrogen atoms, and wherein the heterocyclyl or heteroaryl rings may be optionally substituted with 1 or 2 groups, which may be the same or different, represented by R⁹.

Even more preferably, R⁸ is halogen, C₁-C₃alkyl, C₁-C₃alkoxy, or C₁-C₃haloalkyl; or
any two adjacent R⁸ groups together with the carbon atoms to which they are attached, may form a 5- or 6-membered heterocyclyl ring, comprising 1 or 2 oxygen atoms, and wherein the heterocyclyl ring may be optionally substituted with 1 or 2 groups, which may be the same or different, represented by R⁹.

More preferably still, R⁸ is chloro, bromo, fluoro, methyl, methoxy, or trifluoromethyl; or any two adjacent R⁸ groups together with the carbon atoms to which they are attached, may form a 5-membered heterocyclyl ring, comprising 2 oxygen atoms, and wherein the heterocyclyl ring is substituted with 2 fluoro groups.

In one set of embodiments, R⁸ is halogen, C₁-C₃alkyl, C₁-C₃alkoxy, C₁-C₃haloalky, or C₁-C₃haloalkoxy; or difluoromethoxy; or any two adjacent R⁸ groups together with the carbon atoms to which they are attached, may form a 5-membered heterocyclyl ring, comprising 2 oxygen atoms, and wherein the heterocyclyl ring is substituted with 2 fluoro groups.

In another set of embodiments, R⁸ is halogen, methyl, methoxy, difluoromethyl, or difluoromethoxy; or any two adjacent R⁸ groups together with the carbon atoms to which they are attached, may form a 5-membered heterocyclyl ring, comprising 2 oxygen atoms, and wherein the heterocyclyl ring is substituted with 2 fluoro groups.

R⁹ is halogen, C₁-C₃alkyl, C₁-C₃haloalkyl, or C₁-C₃alkoxy. Preferably, R⁹ is halogen, cyano, methyl or methoxy. More preferably, R⁹ is halogen, methyl or methoxy. More preferably still, R⁹ is halogen. Even more preferably still, R⁹ is fluoro.

R¹⁰ and R¹¹ are each independently selected from hydrogen and C₁-C₃alkyl. Preferably, R¹⁰ and R¹¹ are each independently selected from hydrogen, methyl, and ethyl. In one set of embodiments, R¹⁰ and R¹¹ are both methyl.

R¹² is halogen, cyano, C₁-C₃alkyl, or C₁-C₃alkoxy. Preferably, R¹² is bromo, chloro, fluoro, cyano, methyl or methoxy.

n is 0, 1 or 2. In one set of embodiments, n is 0 or 2. In another set of embodiments n is 0. In a further set of embodiments n is 1. In a still further set of embodiments, n is 2.

In a compound of formula (I) according to the present invention, preferably:
X is O, NR⁷;
R¹ is heteroaryl wherein the heteroaryl moiety is a 5- or 6-membered aromatic ring which comprises 1 or 2 heteroatoms individually selected from N, O and S, and wherein the heteroaryl moiety is optionally substituted with 1, 2, or 3 groups, which may be the same or different, represented by R⁸;
R² is bromo or cyano;
R³ is hydrogen or C₁-C₃alkyl;
R⁴, R⁵, and R⁶ are all hydrogen;
R⁷ is hydrogen, C₁-C₃alkyl; and
R⁸ is halogen, cyano, C₁-C₆alkyl, C₁-C₆alkoxy, C₁-C₆haloalkyl, C₁-C₆haloalkoxy, C₁-C₆alkylsulfanyl, or C₁-C₆alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the aryl, heterocyclyl, or heteroaryl rings may be optionally substituted with 1, 2 or 3 groups, which may be the same or different, represented by R⁹; and
R⁹ is halogen.

In another set of embodiments, X is O;
R¹ is heteroaryl, wherein the heteroaryl moiety is a 6-membered aromatic monocyclic ring comprising 1 or 2 nitrogen atoms, and wherein the heteroaryl moiety is optionally substituted with 1 or 2 groups, which may be the same or different, represented by R⁸;
R² is bromo or cyano;
R³ is hydrogen, methyl, or ethyl;
R⁴, R⁵, and R⁶ are all hydrogen;
R⁸ is halogen, methyl, methoxy, or trifluoromethyl; or
any two adjacent R⁸ groups together with the carbon atoms to which they are attached, may form a 5-membered heterocyclyl ring, comprising 2 oxygen atoms, and wherein the heterocyclyl ring is substituted with 2 fluoro groups.

In a further set of embodiments, X is O;
R¹ is pyridyl or pyrimidinyl, wherein each pyridyl or pyrimidinyl moiety is optionally substituted with 1 or 2 groups, which may be the same or different, represented by R⁸;
R² is bromo, cyano, acetyl, or N-methoxy-C-methyl-carbonimidoyl;
R³ is hydrogen or ethyl;
R⁴, R⁵, and R⁶ are all hydrogen;
R⁸ is halogen, C₁-C₃alkyl, C₁-C₃alkoxy, C₁-C₃haloalky, or C₁-C₃haloalkoxy; or
any two adjacent R⁸ groups together with the carbon atoms to which they are attached, may form a 5-membered heterocyclyl ring, comprising 2 oxygen atoms, and wherein the heterocyclyl ring is substituted with 2 fluoro groups.

Preferably, the compound of Formula (I) is selected from
5-cyano-4-oxo-1-pyrimidin-5-yl-cinnoline-3-carboxylic acid (compound 1);
ethyl 5-bromo-1-(2-chloro-4-pyridyl)-4-oxo-cinnoline-3-carboxylate (compound 3);
ethyl 5-bromo-1-(6-methoxy-3-pyridyl)-4-oxo-cinnoline-3-carboxylate (compound 4);
ethyl 5-bromo-1-(5-chloro-3-pyridyl)-4-oxo-cinnoline-3-carboxylate (compound 5);
ethyl 5-bromo-1-(2-methoxy-4-pyridyl)-4-oxo-cinnoline-3-carboxylate (compound 9);
ethyl 5-bromo-1-(2-bromo-4-pyridyl)-4-oxo-cinnoline-3-carboxylate (compound 10);
ethyl 5-cyano-1-(6-fluoro-3-pyridyl)-4-oxo-cinnoline-3-carboxylate (compound 11);
ethyl 5-bromo-1-(6-fluoro-3-pyridyl)-4-oxo-cinnoline-3-carboxylate (compound 23);
ethyl 5-bromo-1-(2,2-difluoro-[1,3]dioxolo[4,5-b]pyridin-6-yl)-4-oxo-cinnoline-3-carboxylate (compound 24);
ethyl 5-bromo-1-(6-chloro-5-methyl-3-pyridyl)-4-oxo-cinnoline-3-carboxylate (compound 26);
ethyl 5-bromo-1-(6-methoxy-5-methyl-3-pyridyl)-4-oxo-cinnoline-3-carboxylate (compound 29);
ethyl 5-bromo-1-[6-(difluoromethyl)-3-pyridyl]-4-oxo-cinnoline-3-carboxylate (compound 30);
ethyl 5-bromo-1-(6-methoxypyrimidin-4-yl)-4-oxo-cinnoline-3-carboxylate (compound 32);
ethyl 5-bromo-1-(5-chloro-3-cyano-2-pyridyl)-4-oxo-cinnoline-3-carboxylate (compound 33);
ethyl 5-cyano-1-[6-(difluoromethoxy)-3-pyridyl]-4-oxo-cinnoline-3-carboxylate (compound 35);
ethyl 5-bromo-1-[6-(difluoromethoxy)-3-pyridyl]-4-oxo-cinnoline-3-carboxylate (compound 36);
ethyl 5-acetyl-1-[6-(difluoromethoxy)-3-pyridyl]-4-oxo-cinnoline-3-carboxylate (compound 37);
ethyl 5-bromo-1-[2-(difluoromethoxy)-4-pyridyl]-4-oxo-cinnoline-3-carboxylate (compound 38);
ethyl 5-acetyl-1-[2-(difluoromethoxy)-4-pyridyl]-4-oxo-cinnoline-3-carboxylate (compound 40); and
ethyl 1-[2-(difluoromethoxy)-4-pyridyl]-5-[(Z)-N-methoxy-C-methyl-carbonimidoyl]-4-oxo-cinnoline-3-carboxylate (compound 41).

More preferably, the compound of Formula (I) is selected from
5-cyano-4-oxo-1-pyrimidin-5-yl-cinnoline-3-carboxylic acid (compound 1);
ethyl 5-bromo-1-(2-chloro-4-pyridyl)-4-oxo-cinnoline-3-carboxylate (compound 3);
ethyl 5-bromo-1-(6-methoxy-3-pyridyl)-4-oxo-cinnoline-3-carboxylate (compound 4);
ethyl 5-bromo-1-(5-chloro-3-pyridyl)-4-oxo-cinnoline-3-carboxylate (compound 5);
ethyl 5-bromo-1-(2-bromo-4-pyridyl)-4-oxo-cinnoline-3-carboxylate (compound 10);
ethyl 5-cyano-1-(6-fluoro-3-pyridyl)-4-oxo-cinnoline-3-carboxylate (compound 11);
ethyl 5-bromo-1-(6-fluoro-3-pyridyl)-4-oxo-cinnoline-3-carboxylate (compound 23);
ethyl 5-bromo-1-(2,2-difluoro-[1,3]dioxolo[4,5-b]pyridin-6-yl)-4-oxo-cinnoline-3-carboxylate (compound 24);
ethyl 5-bromo-1-(6-chloro-5-methyl-3-pyridyl)-4-oxo-cinnoline-3-carboxylate (compound 26);
ethyl 5-bromo-1-(6-methoxy-5-methyl-3-pyridyl)-4-oxo-cinnoline-3-carboxylate (compound 29);
ethyl 5-bromo-1-[6-(difluoromethyl)-3-pyridyl]-4-oxo-cinnoline-3-carboxylate (compound 30);
ethyl 5-bromo-1-[6-(difluoromethoxy)-3-pyridyl]-4-oxo-cinnoline-3-carboxylate (compound 36);
ethyl 5-acetyl-1-[6-(difluoromethoxy)-3-pyridyl]-4-oxo-cinnoline-3-carboxylate (compound 37);
ethyl 5-bromo-1-[2-(difluoromethoxy)-4-pyridyl]-4-oxo-cinnoline-3-carboxylate (compound 38);
ethyl 5-acetyl-1-[2-(difluoromethoxy)-4-pyridyl]-4-oxo-cinnoline-3-carboxylate (compound 40); and
ethyl 1-[2-(difluoromethoxy)-4-pyridyl]-5-[(Z)-N-methoxy-C-methyl-carbonimidoyl]-4-oxo-cinnoline-3-carboxylate (compound 41).

Compounds of the invention can be made as shown in the following schemes, in which, unless otherwise stated, the definition of each variable is as defined above for a compound of Formula (I). General methods for the production of compounds of Formula (I) are described below. Unless otherwise stated in the text, X, R¹, R², R³, R⁴, R⁵, and R⁶ are as defined hereinbefore. The starting materials used for the preparation of the compounds of the invention may be purchased from usual commercial suppliers or may be prepared by known methods. The starting materials as well as the intermediates may be purified before use in the next step by state of the art methodologies such as chromatography, crystallisation, distillation and filtration.

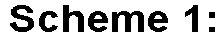

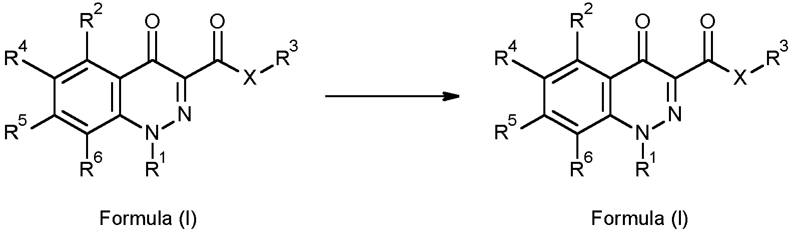

A compound of Formula (I) wherein X is oxygen and R³ is hydrogen may be prepared by hydrolysis of a compound of Formula (I) wherein X is oxygen and R³ is not hydrogen, but any other R³ group as defined above, with a suitable base (such as sodium hydroxide or lithium hydroxide), or with a suitable acid (such as trifluoroacetic acid, hydrochloric acid, formic acid or sulfuric acid), in a suitable solvent (such as methanol, ethanol, dichloromethane, chloroform, ethyl acetate ortetrahydrofuran), with an optional co-solvent (such as water). This is shown in Scheme 1 above. Compounds of Formula (I) may additionally be prepared by methods as described below.

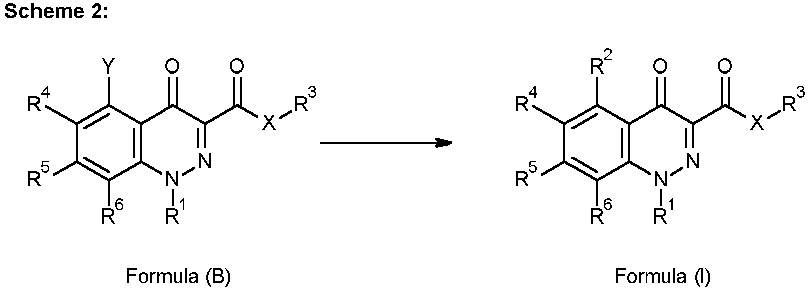

Compounds of Formula (I) may additionally be prepared from a compound of Formula (B) wherein Y is Cl, Br or I. In embodiments of the invention when R² is C₂-C₆alkenyloxy and Y is Br, compounds of Formula (I) may be prepared in a Stille reaction by reaction with a stannane reagent in the presence of a palladium catalyst (such as dichlorobis(triphenylphosphine)palladium(II), tetrakis(triphenylphosphine)palladium) or dichloro(1,1'-bis(diphenylphosphanyl)ferrocene)palladium(II) dichloromethane adduct), with or without a base (such as triethylamine), in a suitable organic solvent (such as toluene, 1,4-dioxane or N,N-dimethylformamide), at an elevated reaction temperature (e.g. 120°C). This is shown in Scheme 2 above.

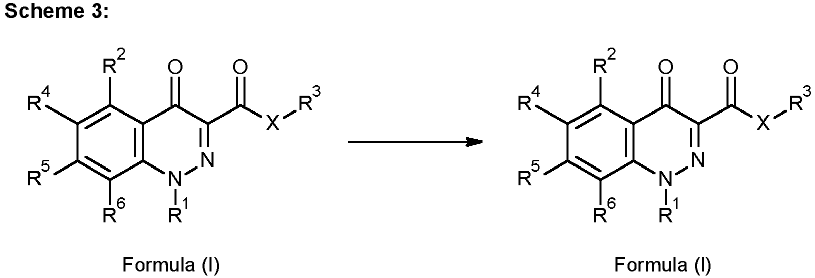

In a subsequent transformation, compounds of Formula (I) wherein R² is C₁-C₆alkylcarbonyl may be prepared from compounds of Formula (I) wherein R² is C₂-C₆alkenyloxy by a hydrolysis reaction. Typically, the reaction is performed by treatment with aqueous acid (such as hydrochloric acid), optionally in a suitable organic solvent (such as acetone, 1,4-dioxane or tetrahydrofuran), and at a suitable temperature (20°C to 60°C). This is shown in Scheme 3 above.

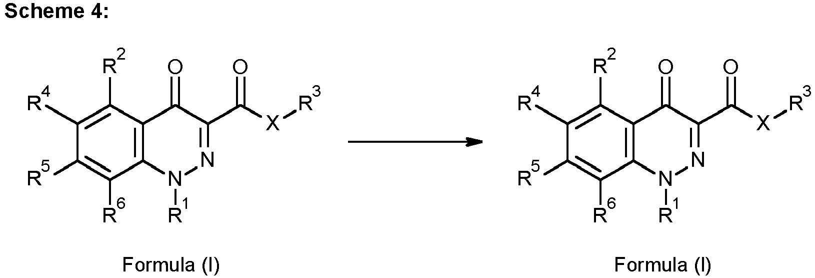

In a further transformation, compounds of Formula (I) wherein R² is -CR¹¹=N-OR¹⁰ may be prepared from compounds of Formula (I) wherein R² is C₁-C₆alkylcarbonyl by a condensation reaction with a suitable hydroxylamine compound. Typically, the reaction is performed with a compound of formula "H₂NOR¹⁰" as either the free base or the hydrochloride salt, with or without the addition of a base (such as sodium acetate, pyridine, or aqueous potassium hydroxide), in a suitable organic solvent (such as ethanol, dimethylsulfoxide, tetrahydrofuran, dimethylether or methanol) with or without additional water at elevated temperature. This is shown in Scheme 4 above.

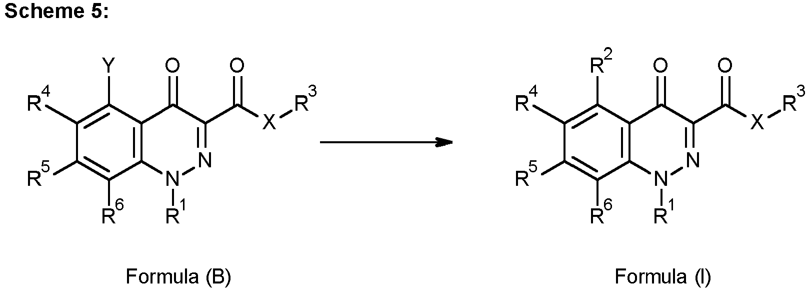

In another transformation, a compound of Formula (B) wherein Y is Br may be converted to a compound of Formula (I) wherein R² is nitrile under Negishi cross-coupling conditions in analogy to known literature conditions. Typically the reaction is performed by reaction of a compound of Formula (B) with dicyanozinc in the presence of a suitable catalyst (such as dichlorobis(triphenylphosphine)palladium(II), tetrakis(triphenylphosphine)palladium), tris(dibenzylideneacetone)dipalladium, dichloro(1,1'-bis(diphenylphosphanyl)ferrocene)palladium(II) dichloromethane adduct), or palladium diacetate, optionally with a ligand (such as 2-dicyclohexylphosphino-2',6'-dimethoxybiphenyl), in a suitable organic solvent (such as dimethylformamide), at elevated temperature. This is shown in Scheme 5 above.

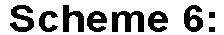

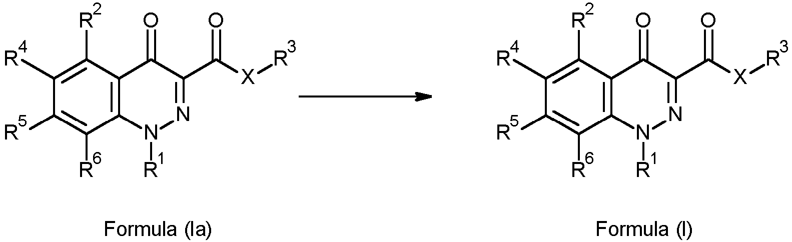

In another transformation, a compound of Formula (Ia) wherein R² is Br or CN and R¹ is hydrogen may be converted to a compound of Formula (I) wherein R¹ is heteroaryl by reaction with a heteroaryl halide under S_{N}Ar conditions in analogy to known literature conditions. Typically, the reaction is performed in the presence of a base (such as potassium carbonate), in an organic solvent (such as dimethylacetamide or N,N-dimethylformamide), at elevated temperature (such as 100°C to 170°C). This is shown in Scheme 6 above.

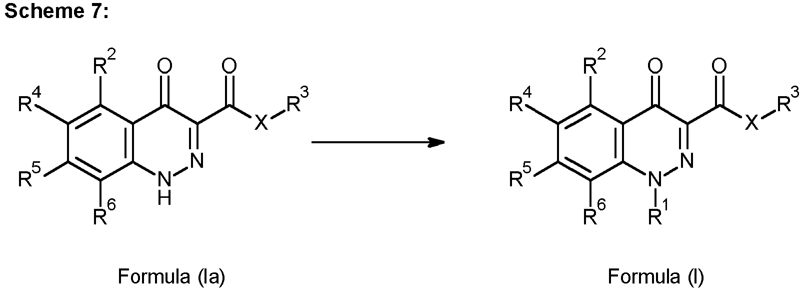

In another transformation, a compound of Formula (la) may be converted to a compound of Formula (I) wherein R¹ is heteroaryl *via* a Chan Lam cross coupling reaction using standard literature conditions. Typically the reaction is performed by reaction of a compound of Formula (la) with an R¹-boronic acid or boroxine in the presence of a suitable copper catalyst (such as copper (II) acetate) optionally in the presence of a base (such as triethylamine, pyridine, N,N-diethylethanamine, or sodium carbonate) in a suitable organic solvent (such as acetonitrile, dimethyl sulfoxide, dichloroethane, or toluene) optionally under a stream of compressed air or in the presence of an additional oxidant (such as boronic acid, pyridine N-oxide, or di-tert-butyl peroxide). This is shown in Scheme 7 above.

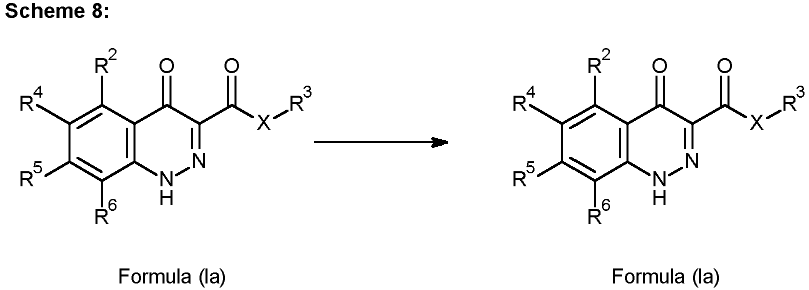

In a different transformation, compounds of Formula (la) wherein R² is Br may be converted to compounds of Formula (la) wherein R² is CN by metal catalysed cross coupling procedures using conditions described in the literature. This is shown in Scheme 8 above.

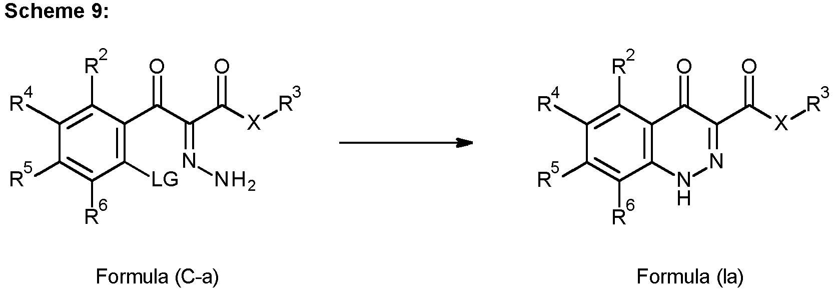

Compounds of Formula (la) may be prepared from compounds of Formula (C-a) where LG is a suitable leaving group (such as F, Cl or Br), by treatment with a base (such as a metal hydride e.g. sodium hydride, or potassium carbonate), or in the absence of base, in a suitable solvent (such as bis(2-methoxyethyl) ether, 1,4-dioxane, tetrahydrofuran or N,N-dimethylformamide), at an elevated temperature (for example 150°C). This is shown in Scheme 9 above.

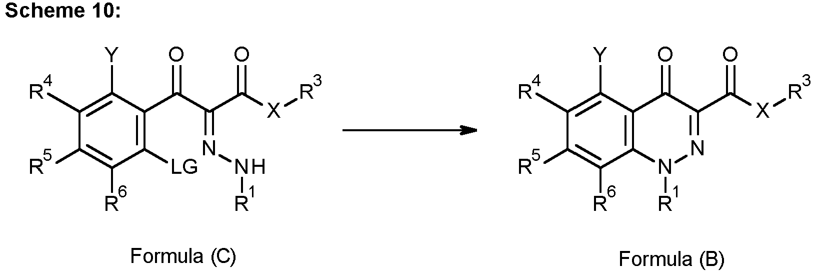

A compound of Formula (B) wherein Y is Br, X is O and LG is a suitable leaving group (such as F, Cl or Br), may be prepared from a compound of Formula (C) by treatment with a base (such as a metal hydride e.g. sodium hydride, or potassium carbonate), in a suitable solvent (such as 1,4-dioxane, tetrahydrofuran or N,N-dimethylformamide), at an elevated temperature (for example 100°C). This is shown in Scheme 10 above.

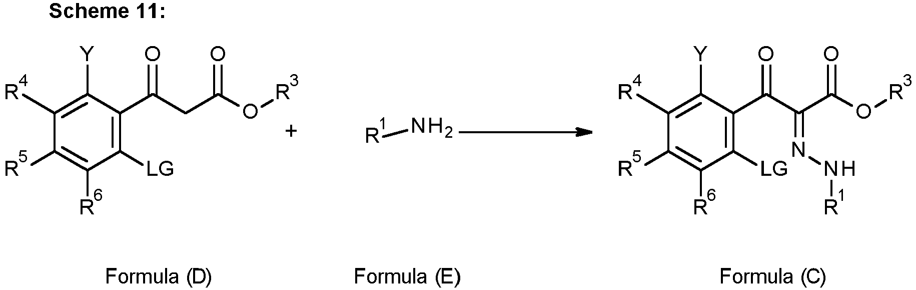

A compound of Formula (C), wherein Y is Br and wherein LG is a suitable leaving group (such as **F,** Cl or Br), may be prepared from reaction of β-keto esters of Formula (D) with an arene diazonium salt. The arene diazonium salts can be prepared *in situ* by diazotisation of anilines of Formula (E) with sodium nitrite in the presence of acid (such as hydrochloric acid), in water followed by reaction with compounds of Formula (D) in the presence of a suitable base (such as sodium or potassium acetate or potassium carbonate), in a suitable solvent (such as water, methanol or ethanol), at temperatures between 0°C and 25°C. Compounds of Formula (E) are commercially available or may be prepared by methods familiar to persons skilled in the art. This is shown in Scheme 11 above.

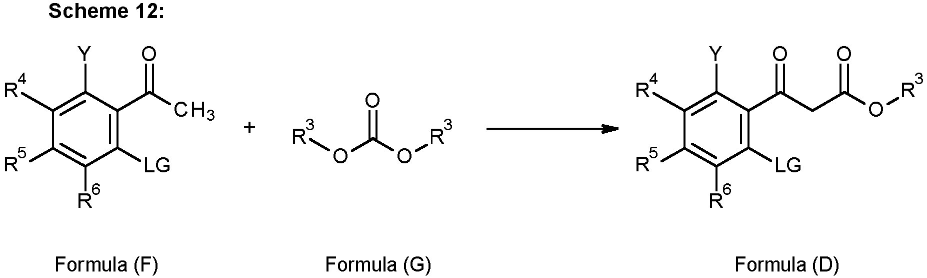

A dicarbonyl compound of Formula (D) wherein Y is Br and wherein LG is a suitable leaving group (such as F, Cl or Br), may be prepared from a methyl ketone compound of Formula (F) and a diester of Formula (G) *via* a Claisen condensation by treatment of the methyl ketone with a suitable base (such as potassium t-butoxide or sodium hydride), in a suitable solvent (such as tetrahydrofuran, N,N-dimethylformamide, toluene or 1,4-dioxane), followed by reaction of the mixture with a carbonate ester (such as dimethylcarbonate or diethylcarbonate), at temperatures between 0°C to 110°C. Compounds of Formula (F) and of Formula (G) are commercially available or may be prepared by methods familiar to persons skilled in the art. This is shown in Scheme 12 above.

The present invention still further provides a method of controlling weeds at a locus said method comprising application to the locus of a weed controlling amount of a composition comprising a compound of Formula (I). Moreover, the present invention may further provide a method of selectively controlling weeds at a locus comprising useful (crop) plants and weeds, wherein the method comprises application to the locus of a weed controlling amount of a composition according to the present invention. 'Controlling' means killing, reducing or retarding growth or preventing or reducing germination. It is noted that the compounds of the present invention show a much improved selectivity compared to know, structurally similar compounds. Generally, the plants to be controlled are unwanted plants (weeds). 'Locus' means the area in which the plants are growing or will grow. The application may be applied to the locus pre-emergence and/or post-emergence of the crop plant. Preferably, the compounds of the present invention are applied to the locus post-emergence of the crop. Some crop plants may be inherently tolerant to herbicidal effects of compounds of Formula (I).

The rates of application of compounds of Formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula (I) according to the invention are generally applied at a rate of from 10 to 2500 g/ha, especially from 25 to 1000 g/ha, more especially from 25 to 250 g/ha.

The application is generally made by spraying the composition, typically by tractor mounted sprayer for large areas, but other methods such as dusting (for powders), drip or drench can also be used.

The term "useful plants" is to be understood as also including useful plants that have been rendered tolerant to herbicides like bromoxynil or classes of herbicides such as, for example, 4-Hydroxyphenylpyruvate dioxygenase (HPPD) inhibitors, ALS inhibitors, for example primisulfuron, prosulfuron and trifloxysulfuron, 5-enol-pyrovyl-shikimate-3-phosphate-synthase (EPSPS) inhibitors, glutamine synthetase (GS) inhibitors or protoporphyrinogen-oxidase (PPO) inhibitors as a result of conventional methods of breeding or genetic engineering. An example of a crop that has been rendered tolerant to imidazolinones, e.g. imazamox, by conventional methods of breeding (mutagenesis) is Clearfield^{®} summer rape (Canola). Examples of crops that have been rendered tolerant to herbicides or classes of herbicides by genetic engineering methods include glyphosate- and glufosinate-resistant maize varieties commercially available under the trade names RoundupReady^{®}, Herculex I^{®} and LibertyLink^{®}.

The term "useful plants" is to be understood as also including useful plants which have been so transformed by the use of recombinant DNA techniques that they are capable of synthesising one or more selectively acting toxins, such as are known, for example, from toxin-producing bacteria, especially those of the genus Bacillus.

Examples of such plants are: YieldGard^{®} (maize variety that expresses a CryIA(b) toxin); YieldGard Rootworm^{®} (maize variety that expresses a CryllIB(b1) toxin); YieldGard Plus^{®} (maize variety that expresses a CryIA(b) and a CryllIB(b1) toxin); Starlink^{®} (maize variety that expresses a Cry9(c) toxin); Herculex I^{®} (maize variety that expresses a CrylF(a2) toxin and the enzyme phosphinothricine N-acetyltransferase (PAT) to achieve tolerance to the herbicide glufosinate ammonium); NuCOTN 33B^{®} (cotton variety that expresses a CryIA(c) toxin); Bollgard I^{®} (cotton variety that expresses a CryIA(c) toxin); Bollgard II^{®} (cotton variety that expresses a CryIA(c) and a CryllA(b) toxin); VIPCOT^{®} (cotton variety that expresses a VIP toxin); NewLeaf^{®} (potato variety that expresses a CryIIIA toxin); NatureGard^{®} Agrisure^{®} GT Advantage (GA21 glyphosate-tolerant trait), Agrisure^{®} CB Advantage (Bt11 corn borer (CB) trait), Agrisure^{®} RW (corn rootworm trait) and Protecta^{®}.

Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crop plants are also to be understood to include those which are obtained by conventional methods of breeding or genetic engineering and contain so-called output traits (e.g. improved storage stability, higher nutritional value and improved flavour).

The compounds of Formula (I) (or compositions comprising such) can be used to control unwanted plants (collectively, 'weeds'). The weeds to be controlled may be both monocotyledonous species, for example *Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum,* and dicotyledonous species, for example *Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium.*

Compounds of Formula (I) may be used in unmodified form or, preferably, together with the adjuvants conventionally employed in the art of formulation to provide herbicidal compositions, using formulation adjuvants, such as carriers, solvents, and surface-active agents (SAA). The invention therefore further provides a herbicidal composition, comprising at least one compound Formula (I) and an agriculturally acceptable carrier and optionally an adjuvant. An agricultural acceptable carrier is for example a carrier that is suitable for agricultural use. Agricultural carriers are well known in the art.

The herbicidal compositions generally comprise from 0.1 to 99 % by weight, especially from 0.1 to 95 % by weight, compounds of Formula I and from 1 to 99.9 % by weight of a formulation adjuvant which preferably includes from 0 to 25 % by weight of a surface-active substance.

The compositions can be chosen from a number of formulation types. These include an emulsion concentrate (EC), a suspension concentrate (SC), a suspo-emulsion (SE), a capsule suspension (CS), a water dispersible granule (WG), an emulsifiable granule (EG), an emulsion, water in oil (EO), an emulsion, oil in water (EW), a micro-emulsion (ME), an oil dispersion (OD), an oil miscible flowable (OF), an oil miscible liquid (OL), a soluble concentrate (SL), an ultra-low volume suspension (SU), an ultra-low volume liquid (UL), a technical concentrate (TK), a dispersible concentrate (DC), a soluble powder (SP), a wettable powder (WP) and a soluble granule (SG). The formulation type chosen in any instance will depend upon the particular purpose envisaged and the physical, chemical, and biological properties of the compound of Formula (I). Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG). Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG). Granules (GR) may be formed either by granulating a mixture of a compound of Formula (I) and one or more powdered solid diluents or carriers, or from pre-formed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent). Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank). Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SAAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water. Microemulsions (ME) may be prepared by mixing water with a blend of one or more solvents with one or more SAAs, to produce spontaneously a thermodynamically stable isotropic liquid formulation. A compound of Formula (I) is present initially in either the water or the solvent/SAA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion. Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product. Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps. Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SAAs), spray additives based on oils, for example certain mineral oils or natural plant oils (such as soy bean and rape seed oil), modified plant oils such as methylated rape seed oil (MRSO), and blends of these with other bio-enhancing adjuvants (ingredients which may aid or modify the action of a compound of Formula (I).

Wetting agents, dispersing agents and emulsifying agents may be SAAs of the cationic, anionic, amphoteric or non-ionic type. Suitable SAAs of the cationic type include quaternary ammonium compounds (for example cetyltrimethyl ammonium bromide), imidazolines and amine salts. Suitable anionic SAAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-isopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates, lignosulphonates and phosphates / sulphates of tristyrylphenols. Suitable SAAs of the amphoteric type include betaines, propionates and glycinates. Suitable SAAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); lecithins and sorbitans and esters thereof, alkyl polyglycosides and tristyrylphenols.

Suitable suspending agents include hydrophilic colloids (such as polysaccharides, polyvinylpyrrolidone or sodium carboxymethylcellulose) and swelling clays (such as bentonite or attapulgite).

The compounds of present invention can also be used in mixture with one or more additional herbicides and/or plant growth regulators. Examples of such additional herbicides or plant growth regulators include acetochlor, acifluorfen (including acifluorfen-sodium), aclonifen, ametryn, amicarbazone, aminopyralid, aminotriazole, atrazine, beflubutamid-M, benquitrione, bensulfuron (including bensulfuron-methyl), bentazone, bicyclopyrone, bilanafos, bipyrazone, bispyribac-sodium, bixlozone, bromacil, bromoxynil, butachlor, butafenacil, carfentrazone (including carfentrazone-ethyl), cloransulam (including cloransulam-methyl), chlorimuron (including chlorimuron-ethyl), chlorotoluron, chlorsulfuron, cinmethylin, clacyfos, clethodim, clodinafop (including clodinafop-propargyl), clomazone, clopyralid, cyclopyranil, cyclopyrimorate, cyclosulfamuron, cyhalofop (including cyhalofop-butyl), 2,4-D (including the choline salt and 2-ethylhexyl ester thereof), 2,4-DB, desmedipham, dicamba (including the aluminium, aminopropyl, bis-aminopropylmethyl, choline, dichloroprop, diglycolamine, dimethylamine, dimethylammonium, potassium and sodium salts thereof) diclosulam, diflufenican, diflufenzopyr, dimethachlor, dimethenamid-P, dioxopyritrione, diquat dibromide, diuron, epyrifenacil, ethalfluralin, ethofumesate, fenoxaprop (including fenoxaprop-P-ethyl), fenoxasulfone, fenpyrazone, fenquinotrione, fentrazamide, flazasulfuron, florasulam, florpyrauxifen (including florpyrauxifen-benzyl), fluazifop (including fluazifop-P-butyl), flucarbazone (including flucarbazone-sodium), flufenacet, flumetsulam, flumioxazin, fluometuron, fomesafen flupyrsulfuron (including flupyrsulfuron-methyl-sodium), fluroxypyr (including fluroxypyr-meptyl), fomesafen, foramsulfuron, glufosinate (including L-glufosinate and the ammonium salts of both), glyphosate (including the diammonium, isopropylammonium and potassium salts thereof), halauxifen (including halauxifen-methyl), haloxyfop (including haloxyfop-methyl), hexazinone, hydantocidin, imazamox (including R-imazamox), imazapic, imazapyr, imazethapyr, indaziflam, iodosulfuron (including iodosulfuron-methyl-sodium), iofensulfuron (including iofensulfuron-sodium), ioxynil, isoproturon, isoxaflutole, lancotrione, MCPA, MCPB, mecoprop-P, mesosulfuron (including mesosulfuron-methyl), mesotrione, metamitron, metazachlor, methiozolin, metolachlor, metosulam, metribuzin, metsulfuron, napropamide, nicosulfuron, norflurazon, oxadiazon, oxasulfuron, oxyfluorfen, paraquat dichloride, pendimethalin, penoxsulam, phenmedipham, picloram, pinoxaden, pretilachlor, primisulfuron-methyl, prometryne, propanil, propaquizafop, propyrisulfuron, propyzamide, prosulfocarb, prosulfuron, pyraclonil, pyraflufen (including pyraflufen-ethyl), pyrasulfotole, pyridate, pyriftalid, pyrimisulfan, pyroxasulfone, pyroxsulam, quinclorac, quinmerac, quizalofop (including quizalofop-P-ethyl and quizalofop-P-tefuryl), rimisoxafen, rimsulfuron, saflufenacil, sethoxydim, simazine, S-metalochlor, sulfentrazone, sulfosulfuron, tebuthiuron, tefuryltrione, tembotrione, terbuthylazine, terbutryn, tetflupyrolimet, thiencarbazone, thifensulfuron, tiafenacil, tolpyralate, topramezone, tralkoxydim, triafamone, triallate, triasulfuron, tribenuron (including tribenuron-methyl), triclopyr, trifloxysulfuron (including trifloxysulfuron-sodium), trifludimoxazin, trifluralin, triflusulfuron, tripyrasulfone, [(E)-[2-(trifluoromethyl)phenyl]methyleneamino] 2,6-bis[(4,6-dimethoxypyrimidin-2-yl)oxy]benzoate, 3-(2-chloro-4-fluoro-5-(3-methyl-2,6-dioxo-4-trifluoromethyl-3,6-dihydropyrimidin-1(2H)-yl)phenyl)-5-methyl-4,5-dihydroisoxazole-5-carboxylic acid ethyl ester,4-hydroxy-1-methoxy-5-methyl-3-[4-(trifluoromethyl)-2-pyridyl]imidazolidin-2-one, 4-hydroxy-1,5-dimethyl-3-[4-(trifluoromethyl)-2-pyridyl]imidazolidin-2-one, 5-ethoxy-4-hydroxy-1-methyl-3-[4-(trifluoromethyl)-2-pyridyl]imidazolidin-2-one, 4-hydroxy-1-methyl-3-[4-(trifluoromethyl)-2-pyridyl]imidazolidin-2-one, 4-hydroxy-1,5-dimethyl-3-[1-methyl-5-(trifluoromethyl)pyrazol-3-yl]imidazolidin-2-one, (4R)1-(5-tert-butylisoxazol-3-yl)-4-ethoxy-5-hydroxy-3-methyl-imidazolidin-2-one, 4-amino-3-chloro-5-fluoro-6-(7-fluoro-1H-indol-6-yl)pyridine-2-carboxylic acid (including agrochemically acceptable esters thereof, for example, methyl 4-amino-3-chloro-5-fluoro-6-(7-fluoro-1H-indol-6-yl)pyridine-2-carboxylate, prop-2-ynyl 4-amino-3-chloro-5-fluoro-6-(7-fluoro-1H-indol-6-yl)pyridine-2-carboxylate and cyanomethyl 4-amino-3-chloro-5-fluoro-6-(7-fluoro-1H-indol-6-yl)pyridine-2-carboxylate), 3-ethylsulfanyl-N-(1,3,4-oxadiazol-2-yl)-5-(trifluoromethyl)-[1,2,4]triazolo[4,3-a]pyridine-8-carboxamide, 3-(isopropylsulfanylmethyl)-N-(5-methyl-1,3,4-oxadiazol-2-yl)-5-(trifluoromethyl)-[1,2,4]triazolo[4,3-a]pyridine-8-carboxamide, 3-(isopropylsulfonylmethyl)-N-(5-methyl-1,3,4-oxadiazol-2-yl)-5-(trifluoromethyl)-[1,2,4]triazolo[4,3-a]pyridine-8-carboxamide, 3-(ethylsulfonylmethyl)-N-(5-methyl-1,3,4-oxadiazol-2-yl)-5-(trifluoromethyl)-[1,2,4]triazolo[4,3-a]pyridine-8-carboxamide, ethyl-2-[[3-[[3-chloro-5-fluoro-6-[3-methyl-2,6-dioxo-4-(trifluoromethyl)pyrimidin-1-yl]-2-pyridyl]oxy]acetate,6-chloro-4-(2,7-dimethyl-1-naphthyl)-5-hydroxy-2-methyl-pyridazin-3-one, tetrahydrofuran-2-ylmethyl (2R)-2-[(4-amino-3,5-dichloro-6-fluoro-2-pyridyl)oxy]propanoate, (2R)-2-[(4-amino-3,5-dichloro-6-fluoro-2-pyridyl)oxy]propanoic acid, tetrahydrofuran-2-ylmethyl 2-[(4-amino-3,5-dichloro-6-fluoro-2-pyridyl)oxy]propanoate, 2-[(4-amino-3,5-dichloro-6-fluoro-2-pyridyl)oxy]propanoic acid, 2-fluoro-N-(5-methyl-1,3,4-oxadiazol-2-yl)-3-[(R)-propylsulfinyl]-4-(trifluoromethyl)benzamide, 2-fluoro-N-(5-methyl-1,3,4-oxadiazol-2-yl)-3-propylsulfinyl-4-(trifluoromethyl)benzamide, (2-fluorophenyl)methyl 6-amino-5-chloro-2-(4-chloro-2-fluoro-3-methoxy-phenyl)pyrimidine-4-carboxylate, 6-amino-5-chloro-2-(4-chloro-2-fluoro-3-methoxy-phenyl)pyrimidine-4-carboxylic acid, 3-(3-chlorophenyl)-6-(5-hydroxy-1,3-dimethylpyrazole-4-carbonyl)-1,5-dimethyl-quinazoline-2,4-dione and [4-[3-(3-chlorophenyl)-1,5-dimethyl-2,4-dioxo-quinazoline-6-carbonyl]-2,5-dimethyl-pyrazol-3-yl] N,N-diethylcarbamate, methyl 2-[(E)-[2-chloro-4-fluoro-5-[3-methyl-2,6-dioxo-4-(trifluoromethyl)pyrimidin-1-yl]phenyl] methyleneamino]oxypropanoate and methyl (2R)-2-[(E)-[2-chloro-4-fluoro-5-[3-methyl-2,6-dioxo-4-(trifluoromethyl)pyrimidin-1-yl]phenyl]methyleneamino] oxypropanoate. The mixing partners of the compound of Formula (I) may also be in the form of esters or salts, as mentioned e.g. in The Pesticide Manual, Sixteenth Edition, British Crop Protection Council, 2012. The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds or mixtures of the present invention can also be used in combination with one or more herbicide safeners. Examples of such safeners include benoxacor, cloquintocet (including cloquintocet-mexyl), cyprosulfamide, dichlormid, fenchlorazole (including fenchlorazole-ethyl), fenclorim, fluxofenim, furilazole, isoxadifen (including isoxadifen-ethyl), mefenpyr (including mefenpyrdiethyl), metcamifen and oxabetrinil. The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048.

Preferably the mixing ratio of compound of Formula (I) to safener is from 100:1 to 1:10, especially from 20:1 to 1:1.

The compounds of Formula (I) are normally used in the form of agrochemical compositions and can be applied to the crop area or plant to be treated, simultaneously or in succession with further compounds. These further compounds can be e.g. fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The term "locus" as used herein means fields in or on which plants are growing, or where seeds of cultivated plants are sown, or where seed will be placed into the soil. It includes soil, seeds, and seedlings, as well as established vegetation.

The term "plants" refers to all physical parts of a plant, including seeds, seedlings, saplings, roots, tubers, stems, stalks, foliage, and fruits.

The term "plant propagation material" is understood to denote generative parts of the plant, such as seeds, which can be used for the multiplication of the latter, and vegetative material, such as cuttings or tubers, for example potatoes. There may be mentioned for example seeds (in the strict sense), roots, fruits, tubers, bulbs, rhizomes, and parts of plants. Germinated plants and young plants which are to be transplanted after germination or after emergence from the soil, may also be mentioned. These young plants may be protected before transplantation by a total or partial treatment by immersion. Preferably "plant propagation material" is understood to denote seeds.

Pesticidal agents referred to herein using their common name are known, for example, from "The Pesticide Manual", 15th Ed., British Crop Protection Council 2009.

The compounds of formula (I) may be used in unmodified form or, preferably, together with the adjuvants conventionally employed in the art of formulation. To this end, they may be conveniently formulated in known manner to emulsifiable concentrates, coatable pastes, directly sprayable or dilutable solutions or suspensions, dilute emulsions, wettable powders, soluble powders, dusts, granulates, and also encapsulations e.g. in polymeric substances. As with the type of the compositions, the methods of application, such as spraying, atomising, dusting, scattering, coating or pouring, are chosen in accordance with the intended objectives and the prevailing circumstances. The compositions may also contain further adjuvants such as stabilizers, antifoams, viscosity regulators, binders or tackifiers as well as fertilizers, micronutrient donors or other formulations for obtaining special effects.

Suitable carriers and adjuvants, e.g., for agricultural use, can be solid or liquid and are substances useful in formulation technology, e.g. natural or regenerated mineral substances, solvents, dispersants, wetting agents, tackifiers, thickeners, binders, or fertilizers. Such carriers are for example described in WO 97/33890.

The compounds of Formula (I) are normally used in the form of compositions and can be applied to the crop area or plant to be treated, simultaneously or in succession with further compounds. These further compounds can be, e.g., fertilizers or micronutrient donors or other preparations, which influence the growth of plants. They can also be selective herbicides or non-selective herbicides as well as insecticides, fungicides, bactericides, nematicides, molluscicides or mixtures of several of these preparations, if desired together with further carriers, surfactants or application promoting adjuvants customarily employed in the art of formulation.

The compound of Formula (I) may be the sole active ingredient of a composition or it may be admixed with one or more additional active ingredients such as a pesticide, fungicide, synergist, herbicide or plant growth regulator where appropriate. An additional active ingredient may, in some cases, result in unexpected synergistic activities.

In general, the formulations include from 0.01 to 90% by weight of active agent, from 0 to 20% agriculturally acceptable surfactant and 10 to 99.99% solid or liquid formulation inerts and adjuvant(s), the active agent consisting of at least the compound of formula (I) together with component (B) and (C), and optionally other active agents, particularly microbiocides or conservatives or the like. Concentrated forms of compositions generally contain in between about 2 and 80%, preferably between about 5 and 70% by weight of active agent. Application forms of formulation may for example contain from 0.01 to 20% by weight, preferably from 0.01 to 5% by weight of active agent. Whereas commercial products will preferably be formulated as concentrates, the end user will normally employ diluted formulations.

The table below illustrates examples of individual compounds of Formula (I) according to the invention:

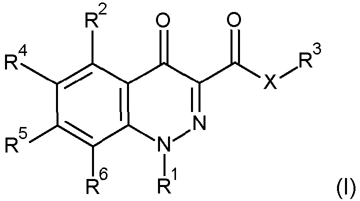

**Table 1: Individual compounds of Formula (I) according to the invention**

| No. | **R¹** | **R²** | **R³** |
|---|---|---|---|
| 001 | 6-(trifluoromethyl)-3-pyridyl | N-methoxy-C-methyl-carbonimidoyl | Et |
| 002 | 6-(trifluoromethyl)-3-pyridyl | N-methoxy-C-methyl-carbonimidoyl | Me |
| 003 | 6-(trifluoromethyl)-3-pyridyl | N-methoxy-C-methyl-carbonimidoyl | H |
| 004 | 6-(trifluoromethyl)-3-pyridyl | 1-ethoxyvinyl | Et |
| 005 | 6-(trifluoromethyl)-3-pyridyl | 1-ethoxyvinyl | Me |
| 006 | 6-(trifluoromethyl)-3-pyridyl | 1-ethoxyvinyl | H |
| 007 | 6-(trifluoromethyl)-3-pyridyl | 1-methoxyvinyl | Et |
| 008 | 6-(trifluoromethyl)-3-pyridyl | 1-methoxyvinyl | Me |
| 009 | 6-(trifluoromethyl)-3-pyridyl | 1-methoxyvinyl | H |
| 010 | 6-(trifluoromethyl)-3-pyridyl | acetyl | Et |
| 011 | 6-(trifluoromethyl)-3-pyridyl | acetyl | Et |
| 012 | 6-(trifluoromethyl)-3-pyridyl | acetyl | Me |
| 013 | 6-(trifluoromethyl)-3-pyridyl | acetyl | H |
| 014 | 6-(trifluoromethyl)-3-pyridyl | methylsulfonyl | Et |
| 015 | 6-(trifluoromethyl)-3-pyridyl | methylsulfonyl | Me |
| 016 | 6-(trifluoromethyl)-3-pyridyl | methylsulfonyl | H |
| 017 | 6-(trifluoromethoxy)-3-pyridyl | N-methoxy-C-methyl-carbonimidoyl | Et |
| 018 | 6-(trifluoromethoxy)-3-pyridyl | N-methoxy-C-methyl-carbonimidoyl | Me |
| 019 | 6-(trifluoromethoxy)-3-pyridyl | N-methoxy-C-methyl-carbonimidoyl | H |
| 020 | 6-(trifluoromethoxy)-3-pyridyl | 1-ethoxyvinyl | Et |
| 021 | 6-(trifluoromethoxy)-3-pyridyl | 1-ethoxyvinyl | Me |
| 022 | 6-(trifluoromethoxy)-3-pyridyl | 1-ethoxyvinyl | H |
| 023 | 6-(trifluoromethoxy)-3-pyridyl | 1-methoxyvinyl | Et |
| 024 | 6-(trifluoromethoxy)-3-pyridyl | 1-methoxyvinyl | Me |
| 025 | 6-(trifluoromethoxy)-3-pyridyl | 1-methoxyvinyl | H |
| 026 | 6-(trifluoromethoxy)-3-pyridyl | acetyl | Et |
| 027 | 6-(trifluoromethoxy)-3-pyridyl | acetyl | Me |
| 028 | 6-(trifluoromethoxy)-3-pyridyl | acetyl | H |
| 029 | 6-(trifluoromethoxy)-3-pyridyl | methylsulfonyl | Et |
| 030 | 6-(trifluoromethoxy)-3-pyridyl | methylsulfonyl | Me |
| 031 | 6-(difluoromethyl)-3-pyridyl | N-methoxy-C-methyl-carbonimidoyl | Et |
| 032 | 6-(difluoromethyl)-3-pyridyl | N-methoxy-C-methyl-carbonimidoyl | Me |
| 033 | 6-(difluoromethyl)-3-pyridyl | N-methoxy-C-methyl-carbonimidoyl | H |
| 034 | 6-(difluoromethyl)-3-pyridyl | 1-ethoxyvinyl | Et |
| 035 | 6-(difluoromethyl)-3-pyridyl | 1-ethoxyvinyl | Me |
| 036 | 6-(difluoromethyl)-3-pyridyl | 1-ethoxyvinyl | H |
| 037 | 6-(difluoromethyl)-3-pyridyl | 1-methoxyvinyl | Et |
| 038 | 6-(difluoromethyl)-3-pyridyl | 1-methoxyvinyl | Me |
| 039 | 6-(difluoromethyl)-3-pyridyl | 1-methoxyvinyl | H |
| 040 | 6-(difluoromethyl)-3-pyridyl | acetyl | Et |
| 041 | 6-(difluoromethyl)-3-pyridyl | acetyl | Me |
| 042 | 6-(difluoromethyl)-3-pyridyl | acetyl | H |
| 043 | 6-(difluoromethyl)-3-pyridyl | methylsulfonyl | Et |
| 044 | 6-(difluoromethyl)-3-pyridyl | methylsulfonyl | Me |
| 045 | 6-(difluoromethyl)-3-pyridyl | methylsulfonyl | H |
| 046 | 6-methoxy-3-pyridyl | N-methoxy-C-methyl-carbonimidoyl | Et |
| 047 | 6-methoxy-3-pyridyl | N-methoxy-C-methyl-carbonimidoyl | Me |
| 048 | 6-methoxy-3-pyridyl | N-methoxy-C-methyl-carbonimidoyl | H |
| 049 | 6-methoxy-3-pyridyl | 1-ethoxyvinyl | Et |
| 050 | 6-methoxy-3-pyridyl | 1-ethoxyvinyl | Me |
| 051 | 6-methoxy-3-pyridyl | 1-ethoxyvinyl | H |
| 052 | 6-methoxy-3-pyridyl | 1-methoxyvinyl | Et |
| 053 | 6-methoxy-3-pyridyl | 1-methoxyvinyl | Me |
| 054 | 6-methoxy-3-pyridyl | 1-methoxyvinyl | H |
| 055 | 6-methoxy-3-pyridyl | acetyl | Et |
| 056 | 6-methoxy-3-pyridyl | acetyl | Me |
| 057 | 6-methoxy-3-pyridyl | acetyl | H |
| 058 | 6-methoxy-3-pyridyl | methylsulfonyl | Et |
| 059 | 6-methoxy-3-pyridyl | methylsulfonyl | Me |
| 060 | 6-methoxy-3-pyridyl | methylsulfonyl | H |
| 061 | 6-chloro-3-pyridyl | N-methoxy-C-methyl-carbonimidoyl | Et |
| 062 | 6-chloro-3-pyridyl | N-methoxy-C-methyl-carbonimidoyl | Me |
| 063 | 6-chloro-3-pyridyl | N-methoxy-C-methyl-carbonimidoyl | H |
| 064 | 6-chloro-3-pyridyl | 1-ethoxyvinyl | Et |
| 065 | 6-chloro-3-pyridyl | 1-ethoxyvinyl | Me |
| 066 | 6-chloro-3-pyridyl | 1-ethoxyvinyl | H |
| 067 | 6-chloro-3-pyridyl | 1-methoxyvinyl | Et |
| 068 | 6-chloro-3-pyridyl | 1-methoxyvinyl | Me |
| 069 | 6-chloro-3-pyridyl | 1-methoxyvinyl | H |
| 070 | 6-chloro-3-pyridyl | acetyl | Et |
| 071 | 6-chloro-3-pyridyl | acetyl | Me |
| 072 | 6-chloro-3-pyridyl | acetyl | H |
| 073 | 6-chloro-3-pyridyl | methylsulfonyl | Et |
| 074 | 6-chloro-3-pyridyl | methylsulfonyl | Me |
| 075 | 6-chloro-3-pyridyl | methylsulfonyl | H |
| 076 | 6-fluoro-3-pyridyl | N-methoxy-C-methyl-carbonimidoyl | Et |
| 077 | 6-fluoro-3-pyridyl | N-methoxy-C-methyl-carbonimidoyl | Me |
| 078 | 6-fluoro-3-pyridyl | N-methoxy-C-methyl-carbonimidoyl | H |
| 079 | 6-fluoro-3-pyridyl | 1-ethoxyvinyl | Et |
| 080 | 6-fluoro-3-pyridyl | 1-ethoxyvinyl | Me |
| 081 | 6-fluoro-3-pyridyl | 1-ethoxyvinyl | H |
| 082 | 6-fluoro-3-pyridyl | 1-methoxyvinyl | Et |
| 083 | 6-fluoro-3-pyridyl | 1-methoxyvinyl | Me |
| 084 | 6-fluoro-3-pyridyl | 1-methoxyvinyl | H |
| 085 | 6-fluoro-3-pyridyl | acetyl | Et |
| 086 | 6-fluoro-3-pyridyl | acetyl | Me |
| 087 | 6-fluoro-3-pyridyl | acetyl | H |
| 088 | 6-fluoro-3-pyridyl | methylsulfonyl | Et |
| 089 | 6-fluoro-3-pyridyl | methylsulfonyl | Me |
| 090 | 6-fluoro-3-pyridyl | methylsulfonyl | H |
| 091 | 5-(trifluoromethyl)-2-pyridyl | N-methoxy-C-methyl-carbonimidoyl | Et |
| 092 | 5-(trifluoromethyl)-2-pyridyl | N-methoxy-C-methyl-carbonimidoyl | Me |
| 093 | 5-(trifluoromethyl)-2-pyridyl | N-methoxy-C-methyl-carbonimidoyl | H |
| 094 | 5-(trifluoromethyl)-2-pyridyl | 1-ethoxyvinyl | Et |
| 095 | 5-(trifluoromethyl)-2-pyridyl | 1-ethoxyvinyl | Me |
| 096 | 5-(trifluoromethyl)-2-pyridyl | 1-ethoxyvinyl | H |
| 097 | 5-(trifluoromethyl)-2-pyridyl | 1-methoxyvinyl | Et |
| 098 | 5-(trifluoromethyl)-2-pyridyl | 1-methoxyvinyl | Me |
| 099 | 5-(trifluoromethyl)-2-pyridyl | 1-methoxyvinyl | H |
| 100 | 5-(trifluoromethyl)-2-pyridyl | acetyl | Et |
| 101 | 5-(trifluoromethyl)-2-pyridyl | acetyl | Me |
| 102 | 5-(trifluoromethyl)-2-pyridyl | acetyl | H |
| 103 | 5-(trifluoromethyl)-2-pyridyl | methylsulfonyl | Et |
| 104 | 5-(trifluoromethyl)-2-pyridyl | methylsulfonyl | Me |
| 105 | 5-(trifluoromethyl)-2-pyridyl | methylsulfonyl | H |
| 106 | 5-chloro-2-pyridyl | N-methoxy-C-methyl-carbonimidoyl | Et |
| 107 | 5-chloro-2-pyridyl | N-methoxy-C-methyl-carbonimidoyl | Me |
| 108 | 5-chloro-2-pyridyl | N-methoxy-C-methyl-carbonimidoyl | H |
| 109 | 5-chloro-2-pyridyl | 1-ethoxyvinyl | Et |
| 110 | 5-chloro-2-pyridyl | 1-ethoxyvinyl | Me |
| 111 | 5-chloro-2-pyridyl | 1-ethoxyvinyl | H |
| 112 | 5-chloro-2-pyridyl | 1-methoxyvinyl | Et |
| 113 | 5-chloro-2-pyridyl | 1-methoxyvinyl | Me |
| 114 | 5-chloro-2-pyridyl | 1-methoxyvinyl | H |
| 115 | 5-chloro-2-pyridyl | acetyl | Et |
| 116 | 5-chloro-2-pyridyl | acetyl | Me |
| 117 | 5-chloro-2-pyridyl | acetyl | H |
| 118 | 5-chloro-2-pyridyl | methylsulfonyl | Et |
| 119 | 5-chloro-2-pyridyl | methylsulfonyl | Me |
| 120 | 5-chloro-2-pyridyl | methylsulfonyl | H |
| 121 | 5-(trifluoromethyl)-3-thienyl | N-methoxy-C-methyl-carbonimidoyl | Et |
| 122 | 5-(trifluoromethyl)-3-thienyl | N-methoxy-C-methyl-carbonimidoyl | Me |
| 123 | 5-(trifluoromethyl)-3-thienyl | N-methoxy-C-methyl-carbonimidoyl | H |
| 124 | 5-(trifluoromethyl)-3-thienyl | 1-ethoxyvinyl | Et |
| 125 | 5-(trifluoromethyl)-3-thienyl | 1-ethoxyvinyl | Me |
| 126 | 5-(trifluoromethyl)-3-thienyl | 1-ethoxyvinyl | H |
| 127 | 5-(trifluoromethyl)-3-thienyl | 1-methoxyvinyl | Et |
| 128 | 5-(trifluoromethyl)-3-thienyl | 1-methoxyvinyl | Me |
| 129 | 5-(trifluoromethyl)-3-thienyl | 1-methoxyvinyl | H |
| 130 | 5-(trifluoromethyl)-3-thienyl | acetyl | Et |
| 131 | 5-(trifluoromethyl)-3-thienyl | acetyl | Me |
| 132 | 5-(trifluoromethyl)-3-thienyl | acetyl | H |
| 133 | 5-(trifluoromethyl)-3-thienyl | methylsulfonyl | Et |
| 134 | 5-(trifluoromethyl)-3-thienyl | methylsulfonyl | Me |
| 135 | 5-(trifluoromethyl)-3-thienyl | methylsulfonyl | H |
| 136 | 1-methyl-5-(trifluoromethyl)pyrazol-3-yl | N-methoxy-C-methyl-carbonimidoyl | Et |
| 137 | 1-methyl-5-(trifluoromethyl)pyrazol-3-yl | N-methoxy-C-methyl-carbonimidoyl | Me |
| 138 | 1-methyl-5-(trifluoromethyl)pyrazol-3-yl | N-methoxy-C-methyl-carbonimidoyl | H |
| 139 | 1-methyl-5-(trifluoromethyl)pyrazol-3-yl | 1-ethoxyvinyl | Et |
| 140 | 1-methyl-5-(trifluoromethyl)pyrazol-3-yl | 1-ethoxyvinyl | Me |
| 141 | 1-methyl-5-(trifluoromethyl)pyrazol-3-yl | 1-ethoxyvinyl | H |
| 142 | 1-methyl-5-(trifluoromethyl)pyrazol-3-yl | 1-methoxyvinyl | Et |
| 143 | 1-methyl-5-(trifluoromethyl)pyrazol-3-yl | 1-methoxyvinyl | Me |
| 144 | 1-methyl-5-(trifluoromethyl)pyrazol-3-yl | 1-methoxyvinyl | H |
| 145 | 1-methyl-5-(trifluoromethyl)pyrazol-3-yl | acetyl | Et |
| 146 | 1-methyl-5-(trifluoromethyl)pyrazol-3-yl | acetyl | Me |
| 147 | 1-methyl-5-(trifluoromethyl)pyrazol-3-yl | acetyl | H |
| 148 | 1-methyl-5-(trifluoromethyl)pyrazol-3-yl | methylsulfonyl | Et |
| 149 | 1-methyl-5-(trifluoromethyl)pyrazol-3-yl | methylsulfonyl | Me |
| 150 | 1-methyl-5-(trifluoromethyl)pyrazol-3-yl | methylsulfonyl | H |

Table A-1 provides 150 compounds A-1.001 to A.1.150 of Formula (I) wherein X is oxygen, R⁴, R⁵, and R⁶ are hydrogen, and R¹, R², and R³ are as defined in Table 1.

### Formulation Examples

| Wettable powders | a) | b) | c) |
|---|---|---|---|
| active ingredient [compound of formula (I)] | 25 % | 50 % | 75 % |
| sodium lignosulfonate | 5 % | 5 % | - |
| sodium lauryl sulfate | 3 % | - | 5 % |
| sodium diisobutylnaphthalenesulfonate | - | 6 % | 10 % |
| phenol polyethylene glycol ether | - | 2 % | - |
| (7-8 mol of ethylene oxide) | | | |
| highly dispersed silicic acid | 5 % | 10 % | 10 % |
| Kaolin | 62 % | 27 % | - |

The active ingredient is thoroughly mixed with the adjuvants and the mixture is thoroughly ground in a suitable mill, affording wettable powders that can be diluted with water to give suspensions of the desired concentration.

| Powders for dry seed treatment | a) | b) | c) |
|---|---|---|---|
| active ingredient [compound of formula (I)] | 25 % | 50 % | 75 % |
| light mineral oil | 5 % | 5% | 5 % |
| highly dispersed silicic acid | 5 % | 5% | - |
| Kaolin | 65 % | 40 % | - |
| Talcum | - | | 20 % |

The active ingredient is thoroughly mixed with the adjuvants and the mixture is thoroughly ground in a suitable mill, affording powders that can be used directly for seed treatment.

### Emulsifiable concentrate

| | |
|---|---|
| active ingredient [compound of formula (I)] | 10 % |
| octylphenol polyethylene glycol ether (4-5 mol of ethylene oxide) | 3 % |
| calcium dodecylbenzenesulfonate | 3 % |
| castor oil polyglycol ether (35 mol of ethylene oxide) | 4 % |
| Cyclohexanone | 30 % |
| xylene mixture | 50 % |

Emulsions of any required dilution, which can be used in plant protection, can be obtained from this concentrate by dilution with water.

| Dusts | a) | b) | c) |
|---|---|---|---|
| Active ingredient [compound of formula (I)] | 5 % | 6 % | 4 % |
| talcum | 95 % | - | - |
| Kaolin | - | 94 % | - |
| mineral filler | - | - | 96 % |

Ready-for-use dusts are obtained by mixing the active ingredient with the carrier and grinding the mixture in a suitable mill. Such powders can also be used for dry dressings for seed.

### Extruder granules

| | |
|---|---|
| Active ingredient [compound of formula (I)] | 15 % |
| sodium lignosulfonate | 2 % |
| carboxymethylcellulose | 1 % |
| Kaolin | 82 % |

The active ingredient is mixed and ground with the adjuvants, and the mixture is moistened with water. The mixture is extruded and then dried in a stream of air.

### Coated granules

| | |
|---|---|
| Active ingredient [compound of formula (I)] | 8 % |
| polyethylene glycol (mol. wt. 200) | 3 % |
| Kaolin | 89 % |

The finely ground active ingredient is uniformly applied, in a mixer, to the kaolin moistened with polyethylene glycol. Non-dusty coated granules are obtained in this manner.

### Suspension concentrate

| | |
|---|---|
| active ingredient [compound of formula (I)] | 40 % |
| propylene glycol | 10 % |
| nonylphenol polyethylene glycol ether (15 mol of ethylene oxide) | 6 % |
| Sodium lignosulfonate | 10 % |
| carboxymethylcellulose | 1 % |
| silicone oil (in the form of a 75 % emulsion in water) | 1 % |
| Water | 32 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Flowable concentrate for seed treatment

| | |
|---|---|
| active ingredient [compound of formula (I)] | 40 % |
| propylene glycol | 5 % |
| copolymer butanol PO/EO | 2 % |
| tristyrenephenole with 10-20 moles EO | 2 % |
| 1,2-benzisothiazolin-3-one (in the form of a 20% solution in water) | 0.5 % |
| monoazo-pigment calcium salt | 5 % |
| Silicone oil (in the form of a 75 % emulsion in water) | 0.2 % |
| Water | 45.3 % |

The finely ground active ingredient is intimately mixed with the adjuvants, giving a suspension concentrate from which suspensions of any desired dilution can be obtained by dilution with water. Using such dilutions, living plants as well as plant propagation material can be treated and protected against infestation by microorganisms, by spraying, pouring or immersion.

### Slow Release Capsule Suspension

28 parts of a combination of the compound of formula (I) are mixed with 2 parts of an aromatic solvent and 7 parts of toluene diisocyanate/polymethylene-polyphenylisocyanate-mixture (8:1). This mixture is emulsified in a mixture of 1.2 parts of polyvinyl alcohol, 0.05 parts of a defoamer and 51.6 parts of water until the desired particle size is achieved. To this emulsion a mixture of 2.8 parts 1,6-diaminohexane in 5.3 parts of water is added. The mixture is agitated until the polymerization reaction is completed. The obtained capsule suspension is stabilized by adding 0.25 parts of a thickener and 3 parts of a dispersing agent. The capsule suspension formulation contains 28% of the active ingredients. The medium capsule diameter is 8-15 microns. The resulting formulation is applied to seeds as an aqueous suspension in an apparatus suitable for that purpose.

### Examples

The following non-limiting examples provide specific synthesis methods for representative compounds of the present invention, as referred to in Table 2 below.

### List of Abbreviations

°C = degrees Celsius, Å = angstrom, CDCl₃ = chloroform-d, d = doublet, dd = doublet of doublets, DMSO = dimethylsulfoxide, m = multiplet, M = molar, MHz = megahertz, q = quartet, s = singlet, t = triplet

### Example 1: Synthesis of 5-cyano-4-oxo-1-pyrimidin-5-yl-cinnoline-3-carboxylic acid (Compound 1)

### Step 1: Synthesis of methyl 3-(2-bromo-6-fluoro-phenyl)-3-oxo-propanoate

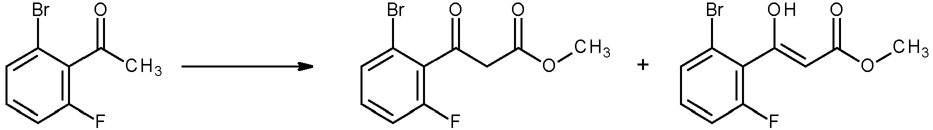

To a stirred solution of 1-(2-bromo-6-fluoro-phenyl)ethanone (5.0 g, 23.0 mmol) and dimethyl carbonate (37.3 g, 406 mmol) in N,N-dimethylformamide (20 mL) under nitrogen and cooled to 0 °C was added portionwise sodium hydride (2.8 g, 69.1 mmol, 60 mass%). The reaction was allowed to warm to room temperature and stirred for 24 hours. The reaction mixture was poured slowly onto ice and acidified to pH3 with concentrated hydrochloric acid. The phases were separated and the aqueous was re-extracted with diethyl ether. The combined organic extracts were dried over magnesium sulfate and evaporated to dryness under reduced pressure. The crude residue was purified by flash chromatography on silica gel using a gradient of 0-30% ethyl acetate in cyclohexane as eluent to give the desired product (mixture of tautomers) as a colourless liquid. Keto Form: ¹H NMR (400 MHz, CDCl₃) δ = 7.46 - 7.36 (m, 1H), 7.33 - 7.28 (m, 1H), 7.15 - 6.98 (m, 1H), 3.98 - 3.90 (m, 2H), 3.79 - 3.61 (m, 3H).

### Step 2: Synthesis of methyl (2Z)-3-(2-bromo-6-fluoro-phenyl)-2-hydrazinylidene-3-oxo-propanoate

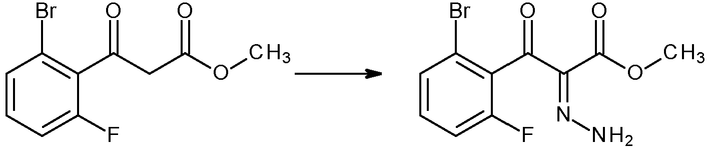

To a cooled (0 °C) solution of methyl 3-(2-bromo-6-fluoro-phenyl)-3-oxo-propanoate (1.00 g, 3.27 mmol) in acetonitrile (10 mL) under an atmosphere of nitrogen was added triethylamine (2.30 mL, 16.4 mmol) followed by dropwise addition of a solution of 4-acetamidobenzene sulfonyl azide (0.785 g, 3.27 mmol) in acetonitrile (5 mL). The reaction mixture was stirred in an ice bath for 15 minutes and then at room temperature for 2 hours. After this time, the resultant reaction mixture was cooled to 0 °C and tributylphosphine (0.826 mL, 3.27 mmol) was added dropwise. The reaction mixture was stirred for 2 hours. The reaction mixture was partitioned between water and ethyl acetate (50 mL). The phases were separated and the aqueous phase was extracted into ethyl acetate (2 x 50 mL). The combined organic extract was washed with brine, dried over anhydrous sodium sulfate and evaporated to dryness under reduced pressure. The crude residue was purified by flash chromatography on silica gel using a gradient of 0-30% ethyl acetate in hexanes as eluent to give the desired product as a light brown solid. ¹H NMR (400 MHz, DMSO-d6) δ = 10.8 (s, 1H), 10.6 (s, 1H), 7.47 (d, 1H), 7.38 - 7.26 (m, 2H), 3.80 (s, 3H).

### Step 3: Synthesis of methyl 5-bromo-4-oxo-1H-cinnoline-3-carboxylate

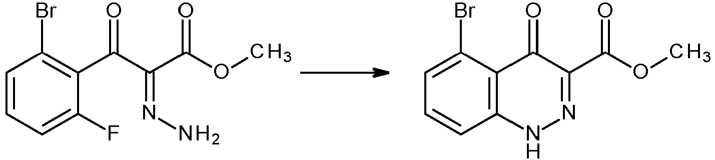

A solution of methyl (2Z)-3-(2-bromo-6-fluoro-phenyl)-2-hydrazinylidene-3-oxo-propanoate (0.700 g, 2.19 mmol) in diglyme (1.5 mL) was heated at 150 °C for 4 hours. After this time, methyl tert-butyl ether (20 mL) was added to the cooled reaction mixture and the mixture was stirred for 20 minutes. The resultant precipitate was filtered and the solid was air-dried to give methyl 5-bromo-4-oxo-1H-cinnoline-3-carboxylate as a brown solid. ¹H NMR (400 MHz, DMSO-d6) δ = 13.8 (s, 1H), 7.71 - 7.64 (m, 3H), 3.83 (s, 3H).

### Step 4: Synthesis of methyl 5-bromo-4-oxo-1-pyrimidin-5-yl-cinnoline-3-carboxylate

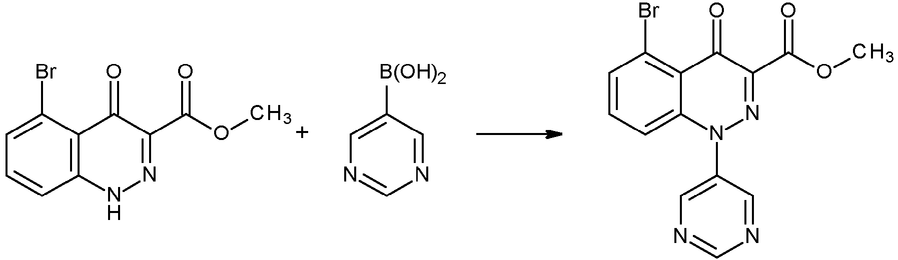

To a mixture of methyl 5-bromo-4-oxo-1H-cinnoline-3-carboxylate (0.700 g, 2.47 mmol) in acetonitrile (30 mL) was added triethylamine (1.43 mL, 9.89 mmol), copper (II) acetate (0.494 g, 2.72 mmol) and pyrimidin-5-ylboronic acid (0.613 g, 4.95 mmol). The resulting solution was heated at 65 °C for 16 hours. The reaction mixture was filtered through diatomaceous earth and partitioned between water and dichloromethane. The aqueous phase was extracted into dichloromethane (x2) and the combined organic extracts were washed sequentially with water, and brine, dried over magnesium sulfate and evaporated to dryness under reduced pressure. The crude residue was purified by flash chromatography on silica gel using a gradient of 0-50% ethyl acetate in hexanes as eluent to give the desired product as a brown solid. ¹H NMR (400 MHz, DMSO-d6) δ = 7.83 (d, 1H), 7.71 - 7.69 (m, 1H), 7.62 - 7.61 (m, 2H), 7.59 (t, 1H), 7.32 (d, 1H), 3.83 (s, 3H).

### Step 5: Synthesis of methyl 5-cyano-4-oxo-1-pyrimidin-5-yl-cinnoline-3-carboxylate

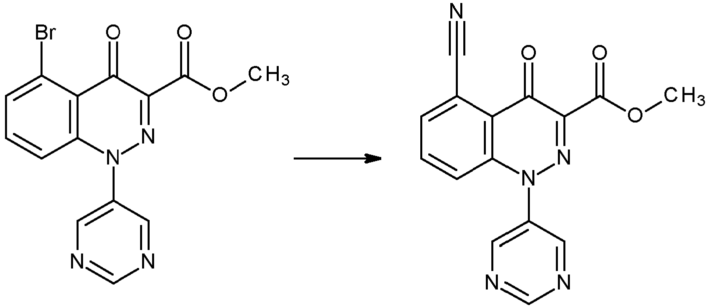

A mixture of methyl 5-bromo-4-oxo-1-pyrimidin-5-yl-cinnoline-3-carboxylate (0.140 g, 0.330 mmol), dicyanozinc (0.077 g, 0.659 mmol) and tetrakis(triphenylphosphine)palladium (0.038 g, 0.033 mmol) in dimethylformamide (5 mL) was purged with argon before being heated under microwave irradiation at 160 °C for 1 hour. The reaction mixture was filtered through diatomaceous earth and concentrated to dryness under reduced pressure. The crude residue was purified by flash chromatography on silica gel using a gradient of 0-100% ethyl acetate in cyclohexane as eluent to give the desired product as a brown solid. ¹H NMR (400 MHz, DMSO-d6) *δ* = 9.46 (s, 1H), 9.20 (s, 2H), 8.14 (d, 1H), 7.91 (t, 1H), 7.72 (d, 1H), 3.87 (s, 3H).

### Step 6: Synthesis of 5-cyano-4-oxo-1-pyrimidin-5-yl-cinnoline-3-carboxylic acid (Compound 1)

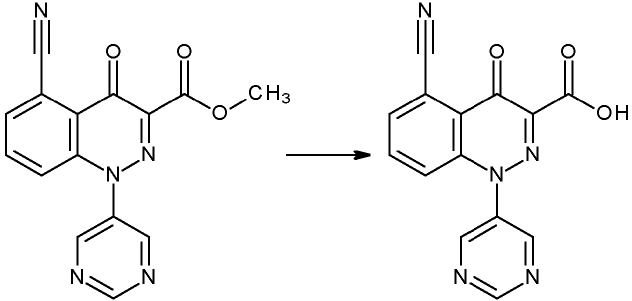

To a solution of methyl 5-cyano-4-oxo-1-pyrimidin-5-yl-cinnoline-3-carboxylate (0.088 g, 0.200 mmol) in tetrahydrofuran (5 mL) was added a solution of lithium hydroxide monohydrate (0.0073 g, 0.17 mmol) in water (1 mL). The resultant solution was stirred at ambient temperature for 1 hour. The solvents were removed under reduced pressure and the residue was diluted with water (20 mL). The pH of the resultant aqueous mixture was adjusted to pH2 by the addition of concentrated hydrochloric acid. The precipitated solid was collected by filtration and dried under reduced pressure to give desired product as a light brown solid. ¹H NMR (400 MHz, DMSO-d6) δ = 9.46 (s, 1H), 9.20 (s, 2H), 8.13 (d, 1H), 7.91 (t, 1H), 7.43 (d, 1H).

### Example 2: Synthesis of methyl 5-bromo-4-oxo-1-[5-(trifluoromethyl)pyrimidin-2-yl]cinnoline-3-carboxylate (Compound 2)

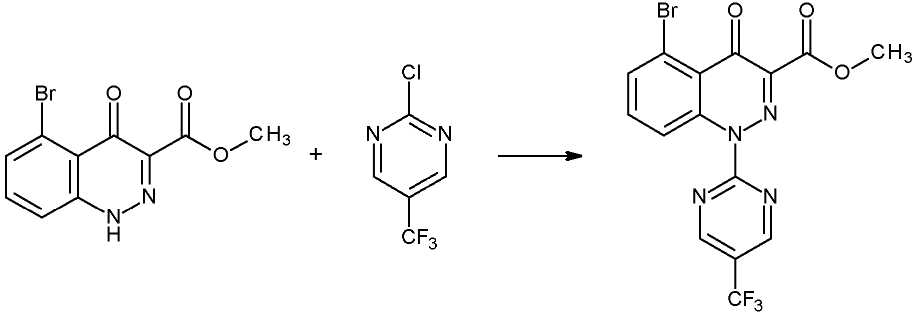

To a solution of methyl 5-bromo-4-oxo-1H-cinnoline-3-carboxylate (0.500 g, 1.77 mmol) in dimethylformamide (5 mL) was added 2-chloro-5-(trifluoromethyl)pyrimidine (0.645 g, 3.53 mmol) and potassium carbonate (0.731 g, 5.30 mmol). The reaction mixture was heated under microwave irradiation at 120 °C for 1 hour. The reaction mixture was filtered through diatomaceous earth and then partitioned between water (50 mL) and dichloromethane (50 mL). The phases were separated and the aqueous phase was extracted into dichloromethane (2 x 50 mL). The combined organic extracts were washed sequentially with water and brine, dried over anhydrous magnesium sulfate, filtered and evaporated to dryness under reduced pressure. The crude residue was purified by flash chromatography on silica gel using a gradient of 0-50% ethyl acetate in hexanes as eluent to give the desired product as a light yellow solid. ¹H NMR (400 MHz, DMSO-d6) δ = 9.57 (s, 2H), 7.88 - 7.85 (m, 2H), 7.63 (t, 1H), 3.87 (s, 3H).

### Example 3: Synthesis of ethyl 5-bromo-1-(2-chloro-4-pyridyl)-4-oxo-cinnoline-3-carboxylate (Compound 3)

### Step 1: Synthesis of ethyl 3-(2-bromo-6-fluoro-phenyl)-3-oxo-propanoate

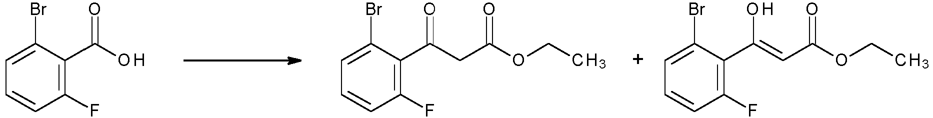

2-bromo-6-fluoro-benzoic acid (0.500 g, 2.3 mmol) was dissolved in tetrahydrofuran (4.6 mL) and stirred with ice-bath cooling under nitrogen. Di(imidazol-1-yl)methanone (0.48 g, 3.0 mmol) was added and the reaction mixture was heated at 65 °C for 0.5 hours. Meanwhile, potassium;3-ethoxy-3-oxo-propanoic acid (1.30 g, 7.6 mmol) and magnesium chloride (0.37 g, 3.9 mmol) were added to tetrahydrofuran (6.9 mL) and stirred as a suspension at room temperature. Triethylamine (0.77 g, 7.6 mmol) was added and the reaction mixture was heated at 65 °C for 3 hours after which time the solution prepared above was added dropwise. On completion of addition the reaction mixture was heated at 50 °C for 18 hours. The cooled reaction mixture was partitioned between aqueous hydrogen chloride solution (2M) and ethyl acetate. The organic phase was washed with brine, dried over anhydrous magnesium sulfate and evaporated to dryness under reduced pressure. The crude residue was purified by flash chromatography on silica gel using a gradient of 0-100% ethyl acetate in cyclohexane as eluent to give the desired product (mixture of tautomers). ¹H NMR (400 MHz, chloroform) δ = 12.29 (s, 1H) enol, 7.45 - 7.39 (m, 1H), 7.32 - 7.23 (m, 1H), 7.14 - 7.07 (m, 1H), 5.28 (s, 1H) enol, 4.29 (q, 1H) enol, 4.19 (q, 1H) keto, 3.91 (d, 1H) keto, 1.34 (t, 1H) enol, 1.24 (t, 2H) keto.

### Step 2: Synthesis of ethyl (2Z)-3-(2-bromo-6-fluoro-phenyl)-2-hydrazinylidene-3-oxo-propanoate

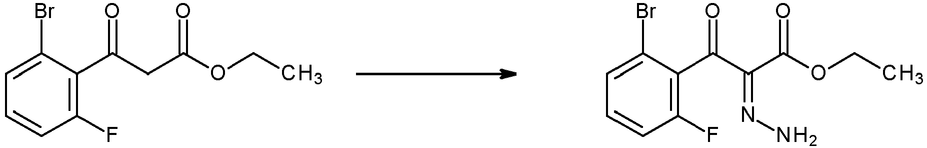

Prepared as for methyl 5-bromo-4-oxo-1H-cinnoline-3-carboxylate from ethyl 3-(2-bromo-6-fluorophenyl)-3-oxo-propanoate (1.0 g, 3.5 mmol) to give ethyl (2Z)-3-(2-bromo-6-fluoro-phenyl)-2-hydrazinylidene-3-oxo-propanoate. ¹H NMR (400 MHz, chloroform) δ ppm 7.34 (d, 1H), 7.20 (td, 1H), 7.03 (t, 1H), 4.39 (q, 2H), 1.40 (t, 3H).

### Step 3: Synthesis of ethyl 5-bromo-4-oxo-1H-cinnoline-3-carboxylate

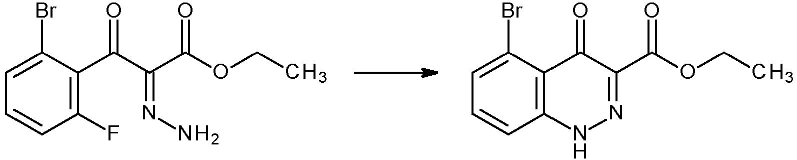

Prepared as for methyl 5-bromo-4-oxo-1H-cinnoline-3-carboxylate using ethyl (2Z)-3-(2-bromo-6-fluorophenyl)-2-hydrazinylidene-3-oxo-propanoate (2.1 g, 6.6 mmol) in bis(2-methoxyethyl) ether (1.5 mL) at 150 °C to afford ethyl 5-bromo-4-oxo-1H-cinnoline-3-carboxylate. ¹H NMR (400 MHz, chloroform) δ ppm 7.77 (d, 1H), 7.67 (d, 1H), 7.56 - 7.49 (m, 1H), 4.52 (q, 2H), 1.44 (t, 3H).

### Step 4: Synthesis of ethyl 5-bromo-4-oxo-1H-cinnoline-3-carboxylate (Compound 3)

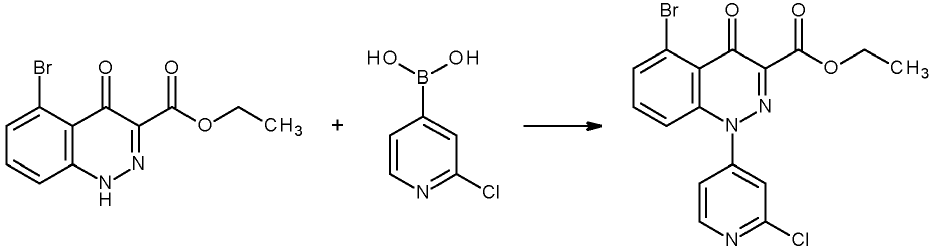

To ethyl 5-bromo-4-oxo-1H-cinnoline-3-carboxylate (0.500 g, 1.7 mmol) in acetonitrile (20 mL) was added (2-chloro-4-pyridyl)boronic acid (0.55 g, 3.5 mmol) and copper (II) acetate (0.36 g, 2.0 mmol). The reaction mixture was heated at 50 °C for 6 hours. The cooled reaction mixture was poured into aqueous hydrogen chloride solution (2 M) and extracted into dichloromethane. The organic extracts were washed with saturated aqueous sodium hydrogen carbonate solution then evaporated to dryness under reduced pressure. The crude residue was purified by flash chromatography on reverse-phase C-18 silica gel using a gradient of 20-85% acetonitrile (+ 0.1 % formic acid) in water (+0.1% formic acid) as eluent to give the desired product as a pale yellow solid. ¹H NMR (400 MHz, chloroform) δ ppm = 8.65 (d, 1H), 7.76 (dd, 1H), 7.57 (d, 1H), 7.50 - 7.41 (m, 2H), 7.28 (dd, 1H), 4.46 (q, 2H), 1.42 (t, 3H).

### Example 4: Synthesis of ethyl 5-bromo-1-(6-methoxy-3-pyridyl)-4-oxo-cinnoline-3-carboxylate (Compound 4)

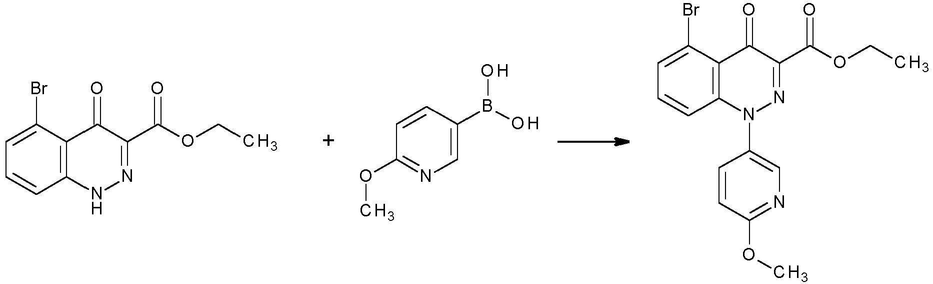

To a suspension of ethyl 5-bromo-4-oxo-1H-cinnoline-3-carboxylate (0.750 g, 2.52 mmol) in acetonitrile (50 mL) was added copper (II) acetate monohydrate (0.555 g, 1.78 mmol), N,N-diethylethanamine (1.02 g, 10.1 mmol) and 4Å molecular sieves (0.061 g). Compressed air was bubbled through the reaction mixture followed by portionwise addition of 6-methoxy-3-pyridyl)boronic acid (0.811 g, 5.30 mmol) at room temperature. The reaction mixture was heated at 60 °C for 0.5 hours under a stream of compressed air and then at 60 °C for 18 hours. The cooled reaction mixture was poured into aqueous hydrogen chloride solution (2M) and extracted into ethyl acetate (x3). The combined organic extracts were washed sequentially with saturated aqueous sodium hydrogen carbonate solution and then brine, dried over anhydrous magnesium sulfate and evaporated to dryness under reduced pressure. The crude residue was purified by flash chromatography on reverse-phase C-18 silica gel using a gradient of 40-70% acetonitrile (+ 0.1 % formic acid) in water (+0.1% formic acid) as eluent to give the desired product as a brown solid. ¹H NMR (400 MHz, chloroform) δ = 8.31 (d, 1H), 7.72 - 7.66 (m, 2H), 7.39 (dd, 1H), 7.11 (dd, 1H), 6.96 (d, 1H), 4.44 (q, 2H), 4.02 (s, 3H), 1.40 (t, 3H).

### Example 5: Synthesis of ethyl 5-cyano-1-(6-methoxy-5-methyl-3-pyridyl)-4-oxo-cinnoline-3-carboxylate (Compound 16)

### Step 1: Synthesis of ethyl 5-cyano-4-oxo-1H-cinnoline-3-carboxylate

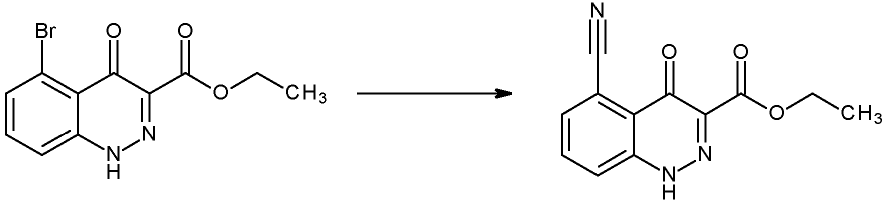

To a solution of ethyl 5-bromo-4-oxo-1H-cinnoline-3-carboxylate (0.500 g, 1.7 mmol) in N,N-dimethylformamide (10 mL) was added zinc cyanide (0.400 g, 3.4 mmol) and tetrakis(triphenyl-λ⁵-phosphanyl)palladium (0.200 g, 0.17 mmol). The reaction mixture was heated under microwave irradiation at 160 °C for 45 minutes. The reaction mixture was partitioned between water and dichloromethane, filtered and phases were separated. The organic phase was evaporated to dryness under reduced pressure and the crude residue was purified by flash chromatography on reverse-phase C-18 silica gel using a gradient of 10-50% acetonitrile (+ 0.1% formic acid) in water (+0.1% formic acid) as eluent to give the desired product as a pale tan solid. ¹H NMR (DMSO-d₆, 400 MHz) δ = 8.02 - 7.92 (m, 1H), 7.88 - 7.74 (m, 2H), 4.43 (q, 2H), 1.42 (t, 3H).

### Step 2: Synthesis of ethyl 5-cyano-1-(6-methoxy-5-methyl-3-pyridyl)-4-oxo-cinnoline-3-carboxylate (Compound 16)

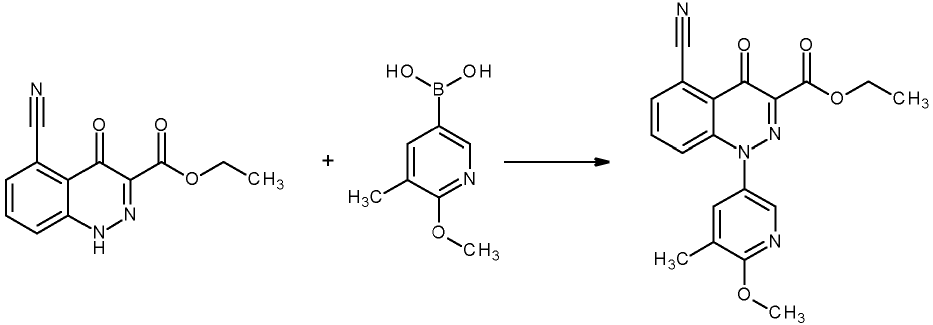

A solution of ethyl 5-cyano-1-(6-methoxy-5-methyl-3-pyridyl)-4-oxo-cinnoline-3-carboxylate (0.200 g, 0.83 mmol) in acetonitrile (6 mL) was added to (6-methoxy-5-methyl-3-pyridyl)boronic acid) followed by acetonitrile (0.34 g, 3.32 mmol) and copper acetate (0.17 g, 0.91 mmol). The reaction mixture was heated at 60 °C for 16 hours under a continuous flow of compressed air. Ethyl acetate (5 mL) and water (5 mL) was added to the cooled reaction mixture and the phases were separated. The aqueous phase was extracted into ethyl acetate (x2). The combined organic extracts were evaporated to dryness under reduced pressure. The crude residue was purified by mass-directed reverse phase HPLC to give desired product. ¹H NMR (500 MHz, chloroform) δ = 8.14 (d, 1H), 7.86 (d, 1H), 7.70 (dd, 1H), 7.50 (d, 1H), 7.43 (d, 1H), 4.47 (q, 2H), 4.06 (s, 3H), 2.29 (s, 3H), 1.41 (t, 3H).

### Example 6: Synthesis of ethyl 5-bromo-1-[2-(difluoromethoxy)-4-pyridyl]-4-oxo-cinnoline-3-carboxylate (Compound 38)

### Step 1: Synthesis of ethyl (2Z)-3-(2-bromo-6-fluoro-phenyl)-2-[[2-(difluoromethoxy)-4-pyridyl]hydrazono]-3-oxo-propanoate

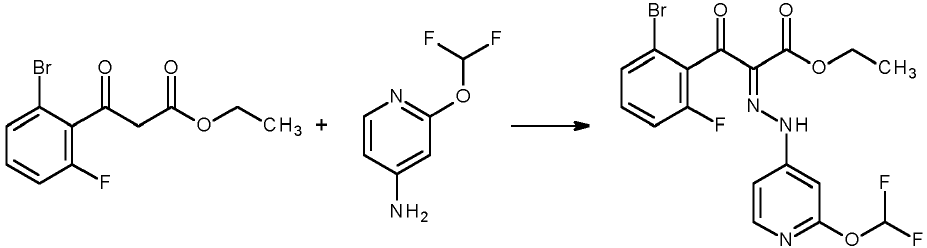

To a stirred solution of 2-(difluoromethoxy)pyridin-4-amine (0.117 g, 0.73 mmol) in dilute aqueous hydrogen chloride solution (2M, 0.5 mL, 0.62 mmol) at 0 °C was added drop-wise a solution of sodium nitrite (0.024 g, 0.34 mmol) in water (2 mL). The resultant reaction mixture was stirred at 0 °C for 2 hours after which time it was added to a stirring solution of ethyl 3-(2-bromo-6-fluoro-phenyl)-3-oxo-propanoate (100 mg, 0.31 mmol) and potassium acetate (0.153 g, 1.56 mmol) at 0 °C. The reaction mixture was stirred at 0 °C for 2 hours before being diluted with cold water and extracted into ethyl acetate (x2). The combined organic extracts were dried over anhydrous sodium sulfate, filtered and evaporated to dryness under reduced pressure to give ethyl (2Z)-3-(2-bromo-6-fluoro-phenyl)-2-[[2-(difluoromethoxy)-4-pyridyl]hydrazono]-3-oxo-propanoate as a crude solid which was used directly in next step without purification.

### Step 2: Synthesis of ethyl 5-bromo-1-[2-(difluoromethoxy)-4-pyridyl]-4-oxo-cinnoline-3-carboxylate (Compound 38)

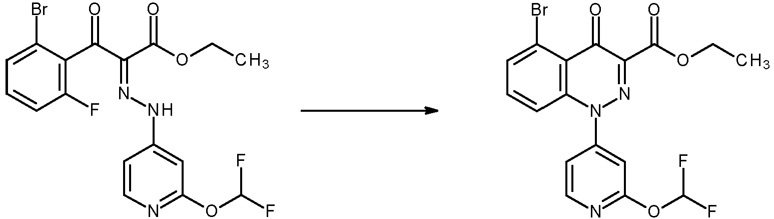

To a stirred solution of ethyl (2Z)-3-(2-bromo-6-fluoro-phenyl)-2-[[2-(difluoromethoxy)-4-pyridyl]hydrazono]-3-oxo-propanoate (200 mg, 0.39 mmol) in N,N-dimethylformamide (4 mL) was added potassium carbonate (0.108 g, 0.78 mmol) at room temperature. The resulting reaction mixture was heated at 100 °C for 4 hours. The cooled reaction mixture was diluted cold water (50 mL) and extracted into ethyl acetate (x2). The combined organic extracts were dried over anhydrous sodium sulfate, filtered and evaporated to dryness under reduced pressure. The crude residue was purified by flash chromatography on silica gel using 20% ethyl acetate in hexanes as eluent to give ethyl 5-bromo-1-[2-(difluoromethoxy)-4-pyridyl]-4-oxo-cinnoline-3-carboxylate as an off-white solid. ¹H NMR (400 MHz, DMSO-d6) δ = 8.57 (d, 1H), 7.78 (t, 1H), 7.81 (d, 1H,) 7.64 - 7.62 (m, 1.5H), 7.61 - 7.58 (dd, 0.5H), 7.52 (d, 1H), 7.35 (dd, 1H), 4.35 (q, 2H), 1.29 (t, 3H).

### Example 7: Synthesis of ethyl 5-acetyl-1-[2-(difluoromethoxy)-4-pyridyl]-4-oxo-cinnoline-3-carboxylate (Compound 40)

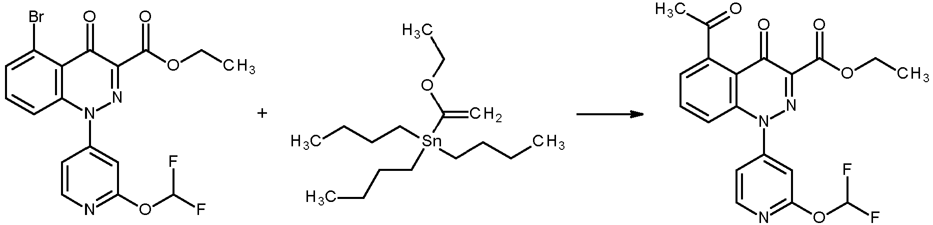

To a stirred solution of ethyl 5-bromo-1-[2-(difluoromethoxy)-4-pyridyl]-4-oxo-cinnoline-3-carboxylate (120 mg, 0.26 mmol) in toluene (20 mL) at room temperature was added tributyl(1-ethoxyvinyl)stannane (187 mg, 0.52 mmol). The resultant reaction mixture was purged with argon for 5 minutes before addition of dichloro palladium; ethylene; triphenylphosphine (19 mg, 0.026 mmol). The resultant reaction mixture was heated in a sealed tube at 110°C for 4 hours. After this time, the cooled reaction mixture was filtered through diatomaceous earth and the filtrate was evaporated to dryness under reduced pressure. The crude residue was purified by flash chromatography on silica gel using 10% ethyl acetate in hexanes as eluent to give ethyl 5-acetyl-1-[2-(difluoromethoxy)-4-pyridyl]-4-oxo-cinnoline-3-carboxylate as an off-white solid. ¹H NMR (400 MHz, DMSO-d6) δ = 8.60 (d, 1H), 7.86 - 7.65 (m, 3H), 7.56 (d, 1H), 7.45 (dd, 2H), 4.35 (q, 2H), 2.46 (s, 3H), 1.30 (t, 3H).

### Example 8: Synthesis of ethyl 1-[2-(difluoromethoxy)-4-pyridyl]-5-[(Z)-N-methoxy-C-methyl-carbonimidoyl]-4-oxo-cinnoline-3-carboxylate (Compound 41)

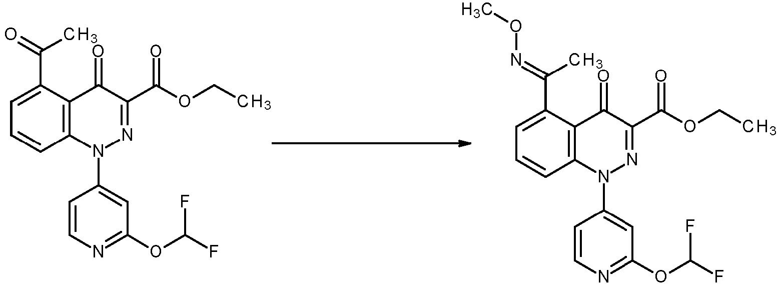

To a stirred solution of ethyl 5-acetyl-1-[2-(difluoromethoxy)-4-pyridyl]-4-oxo-cinnoline-3-carboxylate (50 mg, 0.12 mmol) in a mixture of methanol (3 mL) and water (1 mL) at 0 °C was added sodium acetate (0.015 g, 0.19 mmol) and O-methyl hydroxylamine hydrochloride (0.010 g, 0.12 mmol). The resultant reaction mixture was stirred for 5 minutes before being heated at 80 °C for 6 hours. The cooled reaction mixture was poured onto ice water and neutralized by addition of aqueous hydrogen chloride solution (2M). The mixture was filtered and the filtrate was evaporated under reduced pressure. The crude residue was purified by flash chromatography on reverse-phase C-18 silica gel using a gradient of 0-100% acetonitrile in water to give ethyl 1-[2-(difluoromethoxy)-4-pyridyl]-5-[(Z)-N-methoxy-C-methyl-carbonimidoyl]-4-oxo-cinnoline-3-carboxylate as an off-white solid. ¹H NMR (400 MHz, DMSO-d6) δ = 8.58 (d, 1H), 8.00 (s, 0.5H), 7.82 - 7.76 (m, 1.5H), 7.66 - 7.63 (m, 1H), 7.55 (d, 1H), 7.45 (dd, 1H), 7.34 (dd, 1H), 4.34 (q, 2H), 3.86 (s, 3H), 2.07 (s, 3H), 1.28 (t, 3H).

**Table 2: ¹H NMR Data for selected compounds according to the invention.**

| **Cpd No.** | **Compound Name** | **Structure & ¹H NMR Data** |
|---|---|---|
| 1 | 5-cyano-4-oxo-1-pyrimidin-5-yl-cinnoline-3-carboxylic acid | 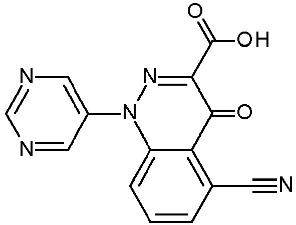 1H NMR (400 MHz, DMSO-d6) δ = 9.46 (s, 1H), 9.20 (s, 2H), 8.13 (d, 1H), 7.91 (t, 1H), 7.43 (d, 1H) |
| 2 | methyl 5-bromo-4-oxo-1-[5-(trifluoromethyl)pyrimidin-2-yl]cinnoline-3-carboxylate | 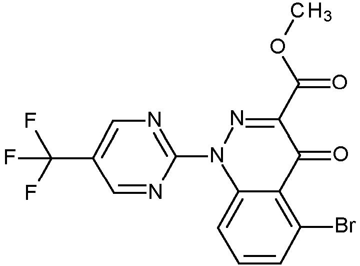 1H NMR (400 MHz, DMSO-d6) δ = 9.57 (s, 2H), 7.88 - 7.85 (m, 2H), 7.63 (t, 1H), 3.87 (s, 3H) |
| 3 | ethyl 5-bromo-1-(2-chloro-4-pyridyl)-4-oxo-cinnoline-3-carboxylate | 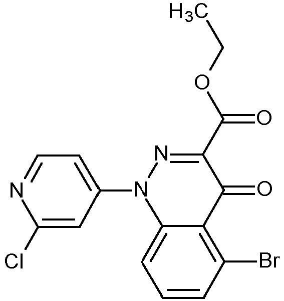 1H NMR (400 MHz, chloroform) δ = 8.65 (d, 1H), 7.76 (dd, 1H), 7.57 (d, 1H), 7.50 - 7.41 (m, 2H), 7.28 (dd, 1H), 4.46 (q, 2H), 1.42 (t, 3H) |
| 4 | ethyl 5-bromo-1-(6-methoxy-3-pyridyl)-4-oxo-cinnoline-3-carboxylate | 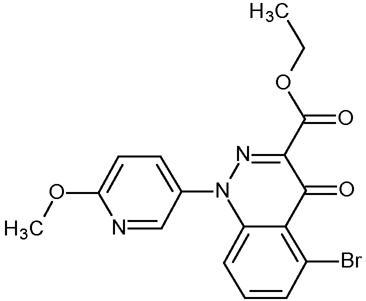 1H NMR (400 MHz, chloroform) δ = 8.31 (d, 1H), 7.72 - 7.66 (m, 2H), 7.39 (dd, 1H), 7.11 (dd, 1H), 6.96 (d, 1H), 4.44 (q, 2H), 4.02 (s, 3H), 1.40 (t, 3H) |
| 5 | ethyl 5-bromo-1-(5-chloro-3-pyridyl)-4-oxo-cinnoline-3-carboxylate | 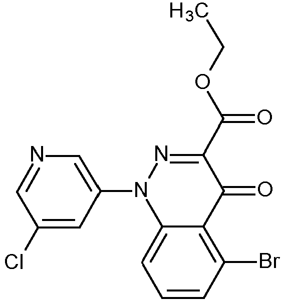 1H NMR (400 MHz, chloroform) δ = 8.77 (d, 1H), 8.69 (d, 1H), 7.92 (t, 1H), 7.73 (dd, 1H), 7.43 (dd, 1H), 7.11 (dd, 1H), 4.45 (q, 2H), 1.41 (t, 3H) |
| 6 | ethyl 5-bromo-1-(2-methoxypyrimidin-5-yl)-4-oxo-cinnoline-3-carboxylate | 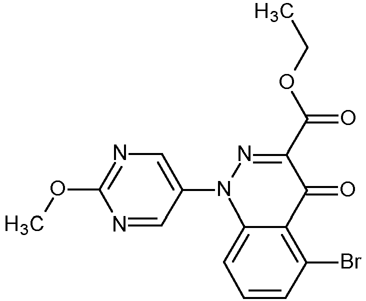 1H NMR (400 MHz, chloroform) δ = 8.68 (s, 2H), 7.78 - 7.71 (m, 1H), 7.43 (dd, 1H), 7.06 (dd, 1H), 4.46 (q, 2H), 4.14 (s, 3H), 1.41 (t, 3H) |
| 7 | ethyl 5-cyano-1-(6-methoxy-3-pyridyl)-4-oxo-cinnoline-3-carboxylate | 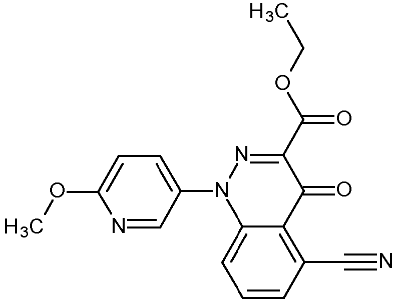 1H NMR (400 MHz, chloroform) δ = 8.34 (d, 1H), 7.87 (dd, 1H), 7.78 - 7.71 (m, 2H), 7.45 (dd, 1H), 6.98 (d, 1H), 4.45 (q, 2H), 4.04 (s, 3H), 1.41 (t, 3H) |
| 8 | ethyl 5-bromo-4-oxo-1-pyrimidin-5-yl-cinnoline-3-carboxylate | 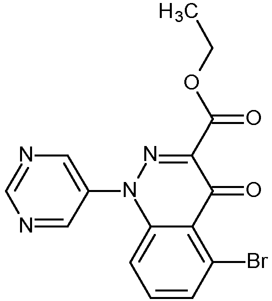 1H NMR (400 MHz, chloroform) δ = 9.40 (s, 1H), 8.97 (s, 2H), 7.76 (dd, 1H), 7.45 (dd, 1H), 7.08 (dd, 1H), 4.46 (q, 2H), 1.41 (t, 3H) |
| 9 | ethyl 5-bromo-1-(2-methoxy-4-pyridyl)-4-oxo-cinnoline-3-carboxylate | 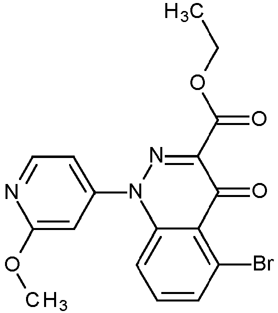 1H NMR (400 MHz, chloroform) δ = 8.39 (d, 1H), 7.71 (dd, 1H), 7.41 (dd, 1H), 7.29 (dd, 1H), 7.04 (dd, 1H), 6.91 (d, 1H), 4.45 (q, 2H), 4.03 (s, 3H), 1.41 (t, 3H) |
| 10 | ethyl 5-bromo-1-(2-bromo-4-pyridyl)-4-oxo-cinnoline-3-carboxylate | 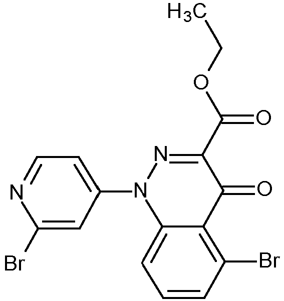 1H NMR (400 MHz, chloroform) δ = 8.63 (d, 1H), 7.76 (dd, 1H), 7.73 (d, 1H), 7.49 - 7.42 (m, 2H), 7.28 (dd, 1H), 4.47 (q, 2H), 1.42 (t, 3H) |
| 11 | ethyl 5-cyano-1-(6-fluoro-3-pyridyl)-4-oxo-cinnoline-3-carboxylate | 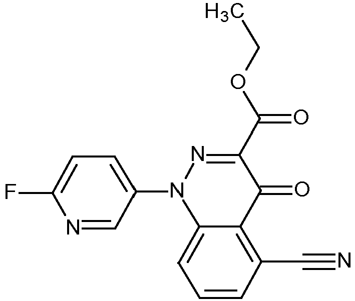 1H NMR (400 MHz, chloroform) δ = 8.47 (d, 1H), 8.08 (ddd, 1H), 7.88 (d, 1H), 7.82 - 7.77 (m, 1H), 7.42 (d, 1H), 7.30 - 7.23 (m, 1H), 4.44 (q, 2H), 1.40 (t, 3H) |
| 12 | ethyl 5-cyano-1-(5-fluoro-6-methoxy-3-pyridyl)-4-oxo-cinnoline-3-carboxylate | 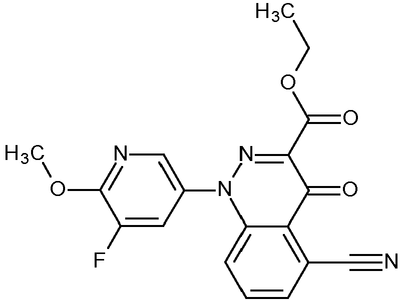 1H NMR (400 MHz, chloroform) δ = 8.15 (d, 1H), 7.88 (dd, 1H), 7.76 (dd, 1H), 7.55 (dd, 1H), 7.45 (dd, 1H), 4.46 (q, 2H), 4.14 (s, 3H), 1.41 (t, 3H) |
| 13 | 5-cyano-1-(6-methoxy-3-pyridyl)-4-oxo-cinnoline-3-carboxylic acid | 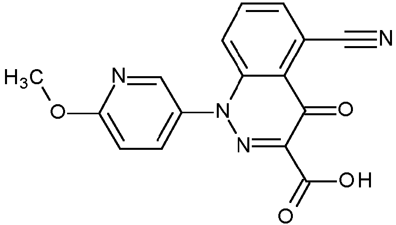 1H NMR (400 MHz, chloroform) δ = 8.35 (d, 1H), 8.08 (dd, 1H), 7.72 (dd, 1H), 7.65 (d, 1H), 7.01 (d, 1H), 6.95 - 6.88 (m, 1H), 4.05 (s, 3H) |
| 14 | ethyl 1-(6-chloro-3-pyridyl)-5-cyano-4-oxo-cinnoline-3-carboxylate | 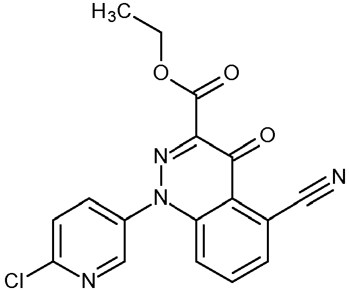 1H NMR (500 MHz, chloroform) δ = 8.61 (d, 1H), 7.90 (d, 1H), 7.85 (dd, 1H), 7.76 (dd, 1H), 7.63 (d, 1H), 7.41 (d, 1H), 4.47 (q, 2H), 1.42 (t, 3H) |
| 15 | ethyl 1-(2-chloro-4-pyridyl)-5-cyano-4-oxo-cinnoline-3-carboxylate | 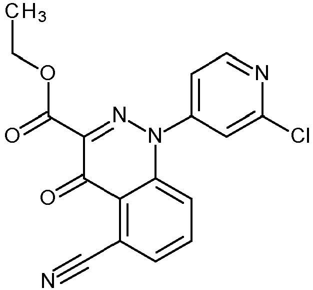 1H NMR (500 MHz, chloroform) δ = 8.69 (d, 1H), 7.92 (d, 1H), 7.79 (t, 1H), 7.62 - 7.57 (m, 2H), 7.45 (dd, 1H), 4.48 (q, 2H), 1.42 (t, 3H) |
| 16 | ethyl 5-cyano-1-(6-methoxy-5-methyl-3-pyridyl)-4-oxo-cinnoline-3-carboxylate | 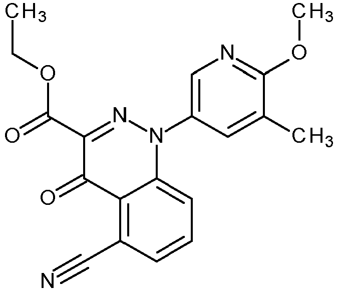 1H NMR (500 MHz, chloroform) δ = 8.14 (d, 1H), 7.86 (d, 1H), 7.70 (dd, 1H), 7.50 (d, 1H), 7.43 (d, 1H), 4.47 (q, 2H), 4.06 (s, 3H), 2.29 (s, 3H), 1.41 (t, 3H) |
| 17 | ethyl 1-(5-chloro-6-methoxy-3-pyridyl)-5-cyano-4-oxo-cinnoline-3-carboxylate | 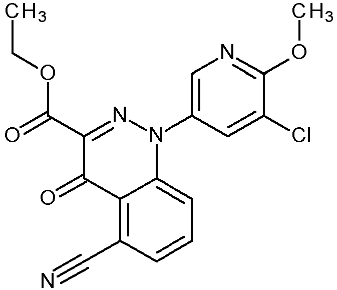 1H NMR (500 MHz, chloroform) δ = 8.24 (d, 1H), 7.89 (dd, 1H), 7.81 (d, 1H), 7.74 (dd, 1H), 7.42 (dd, 1H), 4.47 (q, 2H), 4.13 (s, 3H), 1.42 (t, 3H) |
| 18 | ethyl 5-cyano-1-(2-methoxypyrimidin-5-yl)-4-oxo-cinnoline-3-carboxylate | 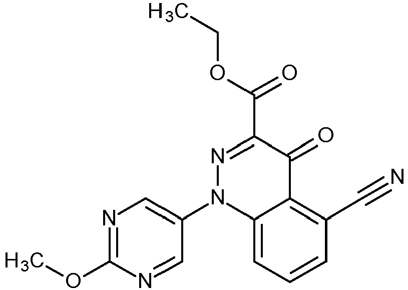 1H NMR (500 MHz, chloroform) δ = 8.70 (s, 2H), 7.91 (dd, 1H), 7.77 (dd, 1H), 7.38 (d, 1H), 4.48 (q, 2H), 4.15 (s, 3H), 1.42 (t, 3H) |
| 19 | ethyl 5-cyano-1-(6-fluoro-5-methyl-3-pyridyl)-4-oxo-cinnoline-3-carboxylate | 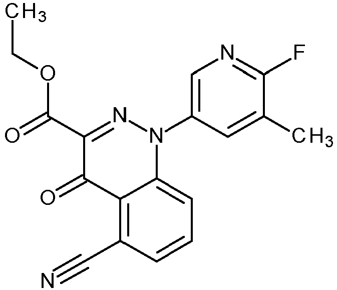 1H NMR (500 MHz, chloroform) δ = 8.24 (s, 1H), 7.89 (d, 1H), 7.79 (dd, 1H), 7.74 (dd, 1H), 7.39 (d, 1H), 4.47 (q, 2H), 2.43 (s, 3H), 1.42 (t, 3H) |
| 20 | ethyl 5-cyano-1-(2-methoxy-4-pyridyl)-4-oxo-cinnoline-3-carboxylate | 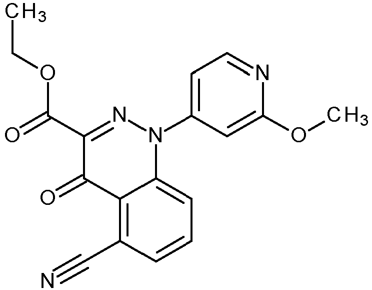 1H NMR (500 MHz, chloroform) δ = 8.42 (d, 1H), 7.88 (d, 1H), 7.73 (dd, 1H), 7.60 (d, 1H), 7.04 (d, 1H), 6.91 (d, 1H), 4.47 (q, 2H), 4.04 (s, 3H), 1.42 (t, 3H) |
| 21 | ethyl 5-cyano-4-oxo-1-[6-(trifluoromethyl)-3-pyridyl]cinnoline-3-carboxylate | 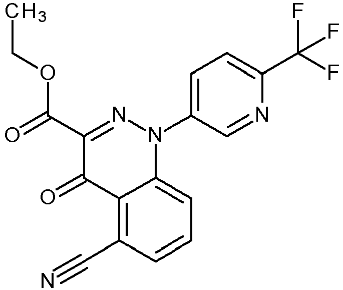 1H NMR (500 MHz, chloroform) δ = 8.94 (d, 1H), 8.11 (dd, 1H), 8.00 (d, 1H), 7.92 (d, 1H), 7.77 (t, 1H), 7.47 - 7.42 (m, 1H), 4.48 (q, 2H), 1.42 (t, 3H) |
| 22 | ethyl 5-bromo-1-(5-fluoro-6-methoxy-3-pyridyl)-4-oxo-cinnoline-3-carboxylate | 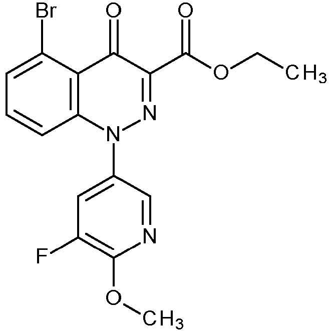 1H NMR (400 MHz, chloroform) δ = 8.12 (d, 1H), 7.72 (d, 1H), 7.50 (dd, 1H), 7.40 (t, 1H), 7.10 (d, 1H), 4.45 (q, 2H), 4.13 (s, 3H), 1.41 (t, 3H) |
| 23 | ethyl 5-bromo-1-(6-fluoro-3-pyridyl)-4-oxo-cinnoline-3-carboxylate | 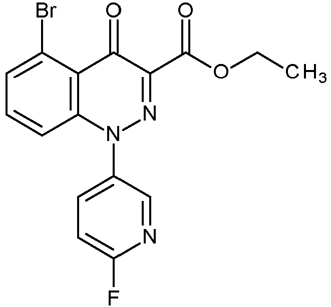 1H NMR (400 MHz, chloroform) δ = 8.42 (d, 1H), 7.95 (ddd, 1H), 7.73 (dd, 1H), 7.41 (dd, 1H), 7.21 (dd, 1H), 7.05 (dd, 1H), 4.46 (q, 2H), 1.41 (t, 3H) |
| 24 | ethyl 5-bromo-1-(2,2-difluoro-[1,3]dioxolo[4,5-b]pyridin-6-yl)-4-oxo-cinnoline-3-carboxylate | 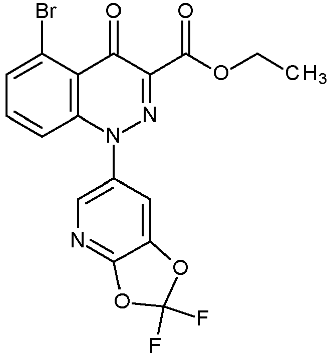 1H NMR (400 MHz, chloroform) δ = 8.16 (d, 1H), 7.74 (d, 1H), 7.55 (d, 1H), 7.43 (t, 1H), 7.09 (d, 1H), 4.44 (q, 2H), 1.40 (t, 3H) |
| 25 | ethyl 5-bromo-4-oxo-1-[6-(trifluoromethyl)-3-pyridyl]cinnoline-3-carboxylate | 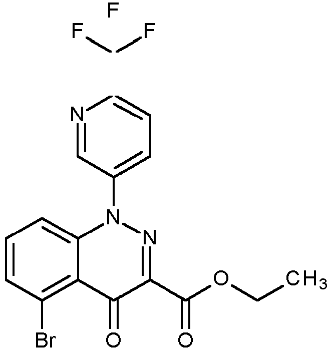 1H NMR (500 MHz, DMSO-d6) δ = 9.10 (d, 1H), 8.47 (dd, 1H), 8.26 (d, 1H), 7.82 (d, 1H), 7.57 (t, 1H), 7.29 (d, 1H), 4.33 (q, 2H), 1.29 (t, 3H) |
| 26 | ethyl 5-bromo-1-(6-chloro-5-methyl-3-pyridyl)-4-oxo-cinnoline-3-carboxylate | 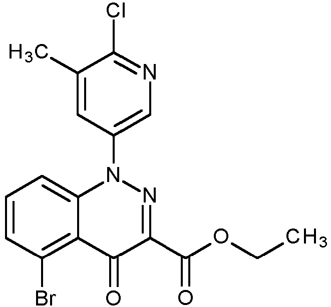 1H NMR (500 MHz, DMSO-d6) δ = 8.59 (d, 1H), 8.20 (d, 1H), 7.80 (d, 1H), 7.57 (t, 1H), 7.25 (d, 1H), 4.32 (q, 2H), 2.44 (s, 3H), 1.29 (t, 3H) |
| 27 | ethyl 1-(6-chloro-5-methyl-3-pyridyl)-5-cyano-4-oxo-cinnoline-3-carboxylate | 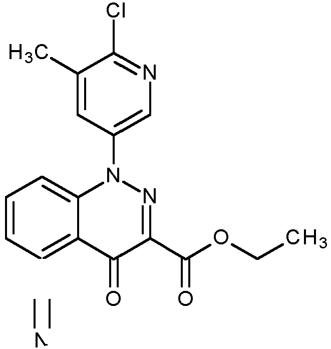 1H NMR (500 MHz, DMSO-d6) δ = 8.60 (d, 1H), 8.21 (d, 1H), 8.11 (d, 1H), 7.89 (dd, 1H), 7.64 (d, 1H), 4.35 (1, 2H), 2.45 (s, 3H), 1.30 (t, 3H) |
| 28 | ethyl 5-bromo-1-(2-methylpyrimidin-5-yl)-4-oxo-cinnoline-3-carboxylate | 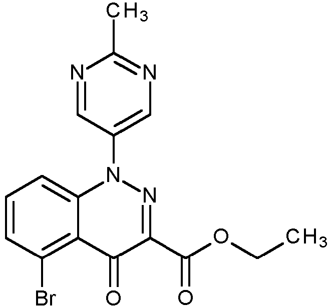 1H NMR (500 MHz, DMSO-d6) δ = 9.05 (s, 2H), 7.82 (d, 1H), 7.57 (t, 1H), 7.29 (d, 1H), 4.33 (q, 2H), 2.78 (s, 3H), 1.29 (t, 3H) |
| 29 | ethyl 5-bromo-1-(6-methoxy-5-methyl-3-pyridyl)-4-oxo-cinnoline-3-carboxylate | 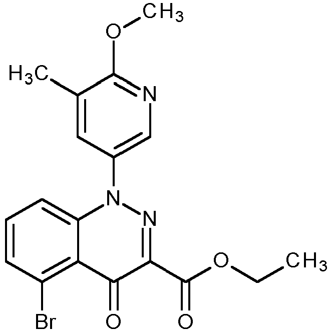 1H NMR (500 MHz, DMSO-d6) δ = 9.05 (s, 2H), 7.82 (d, 1H), 7.57 (t, 1H), 7.29 (d, 1H), 4.33 (q, 2H), 2.78 (s, 3H), 1.29 (t, 3H) |
| 30 | ethyl 5-bromo-1-[6-(difluoromethyl)-3-pyridyl]-4-oxo-cinnoline-3-carboxylate | 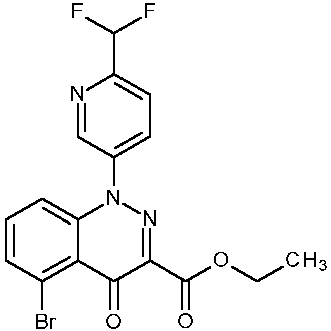 1H NMR (500 MHz, DMSO-d6) δ = 9.01 (d, 1H), 8.37 (dd, 1H), 8.03 (d, 1H), 7.82 (d, 1H), 7.57 (t, 1H), 7.21 (d, 1H), 7.13 (br t, 1H), 4.33 (q, 2H), 1.29 (t, 3H) |
| 31 | ethyl 5-cyano-1-[6-(difluoromethyl)-3-pyridyl]-4-oxo-cinnoline-3-carboxylate | 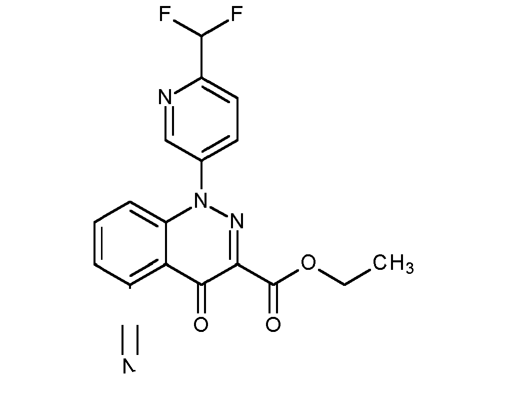 1H NMR (500 MHz, DMSO-d6) δ = 9.02 (d, 1H), 8.38 (dd, 1H), 8.12 (d, 1H), 8.05 (d, 1H), 7.89 (dd, 1H), 7.61 (d, 1H), 7.13 (br t, 1H), 4.35 (q, 2H), 1.30 (t, 3H) |
| 32 | ethyl 5-bromo-1-(6-methoxypyrimidin-4-yl)-4-oxo-cinnoline-3-carboxylate | 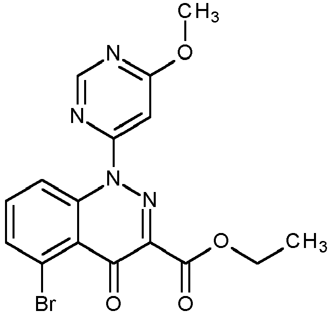 1H NMR (500 MHz, chloroform) δ = 8.78 (s, 1H), 7.94 (d, 1H), 7.74 (d, 1H), 7.45 (t, 1H), 7.13 (s, 1H), 4.47 (q, 2H), 4.10 (s, 3H), 1.42 (t, 3H) |
| 33 | ethyl 5-bromo-1-(5-chloro-3-cyano-2-pyridyl)-4-oxo-cinnoline-3-carboxylate | 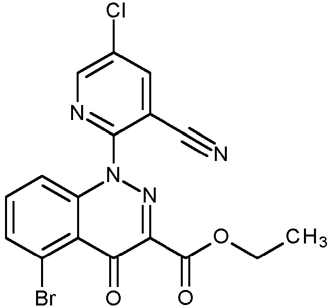 1H NMR (500 MHz, chloroform) δ = 8.75 (d, 1H), 8.27 (d, 1H), 7.76 (d, 1H), 7.43 (t, 1H), 7.26 (d, 1H), 4.46 (q, 2H), 1.41 (t, 3H) |
| 34 | ethyl 5-bromo-1-(5-chloropyrimidin-2-yl)-4-oxo-cinnoline-3-carboxylate | 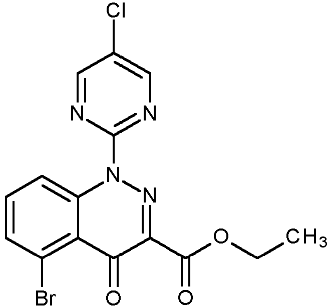 1H NMR (500 MHz, chloroform) δ = 8.90 (s, 2H), 7.74 (d, 1H), 7.51 (d, 1H), 7.42 (t, 1H), 4.46 (q, 2H), 1.41 (t, 3H) |
| 35 | ethyl 5-cyano-1-[6-(difluoromethoxy)-3-pyridyl]-4-oxo-cinnoline-3-carboxylate | 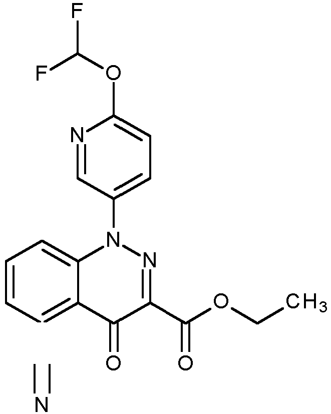 1H NMR (400 MHz, DMSO-d6) δ = 8.67 (d, 1H), 8.28 (dd, 1H), 8.11 (dd, 1H), 7.91 - 7.87 (m, 1H), 7.75 (t, 1H), 7.57 (dd, 1H), 7.42 (d, 1H), 4.35 (q, 2H), 1.30 (t, 3H) |
| 36 | ethyl 5-bromo-1-[6-(difluoromethoxy)-3-pyridyl]-4-oxo-cinnoline-3-carboxylate | 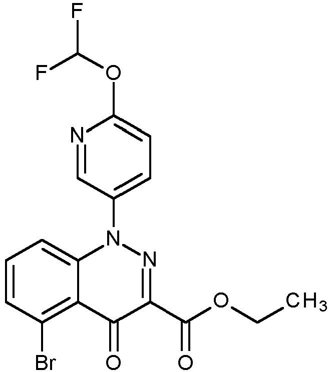 1H NMR (400 MHz, DMSO-d6) δ = 8.62 (s, 1H), 8.28 (dd, 1H), 7.80 (s, 1H), 7.59 (dd, 1.5H), 7.41 (d, 1.5H), 7.18 (dd, 1H), 4.33 (q, 2H), 1.28 (t, 3H) |
| 37 | ethyl 5-acetyl-1-[6-(difluoromethoxy)-3-pyridyl]-4-oxo-cinnoline-3-carboxylate | 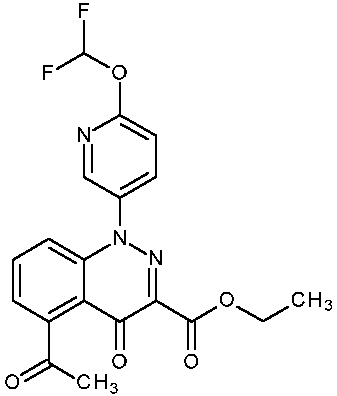 1H NMR (400 MHz, DMSO-d6) δ = 8.65 (d, 1H), 8.31 (dd, 1H), 7.84 - 7.80 (m, 2H), 7.41 (t, 2H), 7.30 (d, 1H), 4.32 (q, 2H), 2.46 (s, 3H), 1.28 (t, 3H) |
| 38 | ethyl 5-bromo-1-[2-(difluoromethoxy)-4-pyridyl]-4-oxo-cinnoline-3-carboxylate | 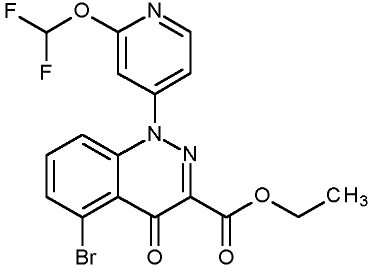 1H NMR (400 MHz, DMSO-d6) δ = 8.57 (d, 1H), 7.78 (t, 1H), 7.81 (d, 1H,) 7.64 - 7.62 (m, 1.5H), 7.61 - 7.58 (dd, 0.5H), 7.52 (d, 1H), 7.35 (dd, 1H), 4.35 (q, 2H), 1.29 (t, 3H) |
| 39 | ethyl 5-cyano-1-[2-(difluoromethoxy)-4-pyridyl]-4-oxo-cinnoline-3-carboxylate | 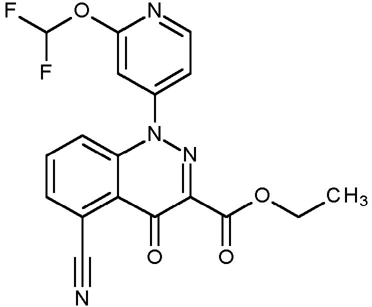 1H NMR (400 MHz, DMSO-d6) δ = 8.60 (d, 1H), 8.13 (dd, 1H), 7.99 - 7.89 (m, 1.5 H), 7.81 (d, 0.5H), 7.75 (dd, 1H), 7.63 (dd, 1H), 7.51 (d, 1H), 4.35 (q, 2H), 1.30 (t, 3H) |
| 40 | ethyl 5-acetyl-1-[2-(difluoromethoxy)-4-pyridyl]-4-oxo-cinnoline-3-carboxylate | 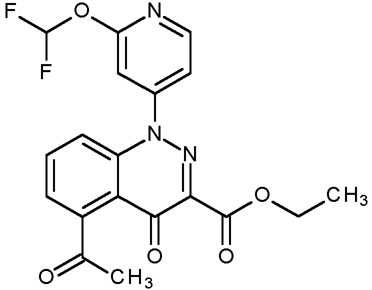 1H NMR (400 MHz, DMSO-d6) δ = 8.60 (d, 1H), 7.86 - 7.65 (m, 3H), 7.56 (d, 1H), 7.45 (dd, 2H), 4.35 (q, 2H), 2.46 (s, 3H), 1.30 (t, 3H) |
| 41 | ethyl 1-[2-(difluoromethoxy)-4-pyridyl]-5-[(Z)-N-methoxy-C-methyl-carbonimidoyl]-4-oxo-cinnoline-3-carboxylate | 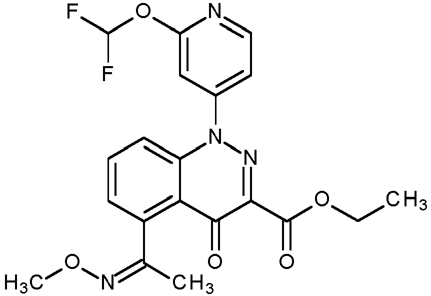 1H NMR (400 MHz, DMSO-d6) δ = 8.58 (d, 1H), 8.00 (s, 0.5H), 7.82 - 7.76 (m, 1.5H), 7.66 - 7.63 (m, 1H), 7.55 (d, 1H), 7.45 (dd, 1H), 7.34 (dd, 1H), 4.34 (q, 2H), 3.86 (s, 3H), 2.07 (s, 3H), 1.28 (t, 3H) |
| 42 | 1-[2-(difluoromethoxy)-4-pyridyl]-5-[(Z)-N-methoxy-C-methyl-carbonimidoyl]-4-oxo-cinnoline-3-carboxylic acid | 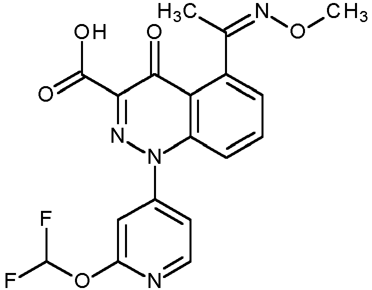 1H NMR (400 MHz, DMSO-d6) δ = 8.57 (d, 1H), 8.00 - 7.75 (m, 2H), 7.64 - 7.61 (m, 1H), 7.51 - 7.50 (m, 2H), 7.31 (d, 1H), 3.86 (s, 3H), 2.07 (s, 3H) |
| 43 | ethyl 5-cyano-1-(2-methylpyrimidin-5-yl)-4-oxo-cinnoline-3-carboxylate | 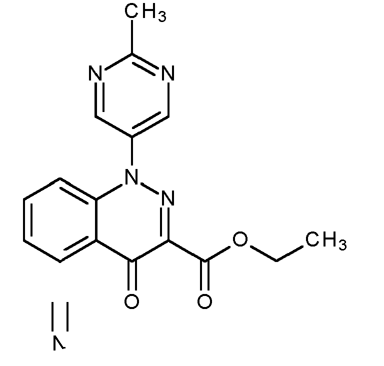 1H NMR (500 MHz, DMSO-d6) δ = 9.05 (s, 2H), 8.12 (d, 1H), 7.90 (dd, 1H), 7.70 (d, 1H), 4.35 (q, 2H), 2.79 (s, 3H), 1.30 (t, 3H) |

### Biological examples

Seeds of a variety of test species are sown in standard soil in pots (*Amaranthus retoflexus* (AMARE), *Echinochloa crus-galli* (ECHCG), *Amaranthus palmeri* (AMAPA), *Setaria faberi* (SETFA), *Zea mays* (ZEAMX), *Ipomoea hederacea* (IPOHE)). After 8 days cultivation under controlled conditions in a glasshouse (at 24 °C /16 °C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 1000 g/ha unless otherwise stated. The test plants are then grown in a glasshouse under controlled conditions in a glasshouse (at 24 °C/16 °C, day/night; 14 hours light; 65 % humidity) and watered twice daily. After 13 days the test is evaluated for the percentage damage caused to the plant. The biological activities are shown in the following table on a five-point scale (5 = 81-100%; 4 = 61-80%; 3=41-60%; 2=21-40%; 1=10-20%; 0=0%; - = not tested).

**TABLE B1: Pre-emergence Test**

| **Cpd No.** | **AMARE** | **ECHCG** | **AMAPA** | **SETFA** | **ZEAMX** | **IPOHE** |
|---|---|---|---|---|---|---|
| 1 | 0 | 2 | 0 | 0 | 0 | 0 |
| 10 | 1 | - | 0 | 0 | 0 | 0 |
| 23 | 0 | 5 | 0 | 4 | 0 | 1 |
| 26 | 1 | 0 | 1 | - | 0 | 0 |
| 30 | 0 | 2 | 0 | - | 0 | 0 |
| 32 | 0 | 1 | 0 | 1 | 0 | 0 |
| 34 | 0 | 0 | 1 | 1 | 0 | 0 |
| 36 | 0 | 0 | 1 | 1 | 1 | 0 |
| 37 | 0 | 4 | 0 | - | 4 | 0 |
| 40 | 0 | 3 | 0 | - | 0 | 0 |
| 41 | 0 | 4 | 0 | - | 1 | 0 |

**TABLE B2: Post-emergence Test**

| **Cpd No.** | **AMARE** | **ECHCG** | **AMAPA** | **SETFA** | **ZEAMX** | **IPOHE** |
|---|---|---|---|---|---|---|
| 1 | 2 | 2 | 1 | 1 | 1 | 1 |
| 3 | 2 | 2 | 1 | 0 | 0 | 0 |
| 4 | 2 | 0 | 1 | 0 | 0 | 0 |
| 5 | 1 | 2 | 1 | 2 | 1 | 1 |
| 8 | 0 | 0 | 0 | 0 | 0 | 0 |
| 9 | 1 | 1 | 1 | 0 | 1 | 0 |
| 10 | 3 | 1 | 3 | 0 | 0 | 0 |
| 11 | 0 | 2 | 0 | 0 | 1 | 0 |
| 22 | 0 | 0 | 0 | 0 | 0 | 0 |
| 23 | 0 | 4 | 0 | 4 | 1 | 0 |
| 24 | 0 | 2 | 0 | 2 | 1 | 0 |
| 26 | 2 | 0 | 1 | 1 | - | 2 |
| 29 | 2 | 1 | 1 | 0 | - | 2 |
| 30 | 1 | 4 | 1 | 0 | - | 2 |
| 32 | 1 | 0 | 1 | 0 | 0 | 1 |
| 33 | 0 | 1 | - | 1 | 0 | 1 |
| 34 | 3 | 0 | 1 | 2 | 1 | 3 |
| 35 | 1 | 0 | 1 | 0 | - | 1 |
| 36 | 2 | 0 | 0 | 1 | 0 | 1 |
| 37 | 1 | 4 | 0 | 1 | - | 2 |
| 38 | 2 | 0 | 1 | 1 | - | 2 |
| 40 | 1 | 4 | 0 | 1 | - | 1 |
| 41 | 1 | 2 | 1 | 1 | - | 1 |

## Claims

1. A compound of Formula (I): 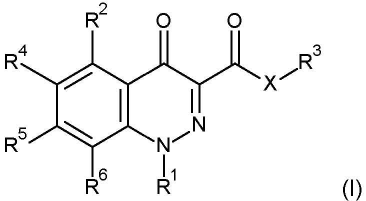 wherein
X is O, NR⁷ or S;
R¹ is heteroaryl, wherein the heteroaryl moiety is a 5- or 6-membered aromatic ring which comprises 1, 2, or 3 heteroatoms individually selected from N, O and S, and wherein the heteroaryl moiety is optionally substituted with 1, 2, 3, or 4 groups, which may be the same or different, represented by R⁸;
R² is halogen, cyano, cyanoC₁-C₆alkyl, C₁-C₆haloalkyl, C₁-C₆alkylcarbonyl, C₂-C₆alkenyl, C₂-C₆alkynyl, C₁-C₆alkoxyC₂-C₆alkenyl, C₂-C₆alkenyloxyC₁-C₆alkyl, -CR¹¹=N-OR¹⁰, nitro, S(O)ₙC₁-C₆alkyl, S(O)ₙC₁-C₆haloalkyl, or S(O)ₙC₃-C₆cycloalkyl;
R³ is hydrogen, C₁-C₆alkyl, C₁-C₆haloalkyl, C₃-C₆cycloalkyl, C₃-C₆cycloalkylC₁-C₆alkyl, C₁-C₆alkoxyC₁-C₆alkyl, C₂-C₆alkenyl, C₂-C₆alkynyl, phenyl, or phenylC₁-C₃alkyl, wherein the phenyl moieties may be optionally substituted with 1, 2, 3, or 4 groups, which may be the same or different, represented by R¹²;
R⁴, R⁵, and R⁶ are each independently selected from hydrogen, halogen, cyano, C₁-C₆alkyl, C₁-C₆alkoxy, C₁-C₆haloalkyl, C₁-C₆haloalkoxy, C₁-C₆alkylsulfanyl, C₁-C₆alkylsulfinyl, and C₁-C₆alkylsulfonyl;
R⁷ is hydrogen, C₁-C₃alkyl, or C₁-C₃alkoxy;
R⁸ is halogen, cyano, C₁-C₆alkyl, C₁-C₆alkoxy, C₁-C₆haloalkyl, C₁-C₆haloalkoxy, C₁-C₆alkylsulfanyl, or C₁-C₆alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the aryl, heterocyclyl, or heteroaryl rings may be optionally substituted with 1, 2, 3 or 4 groups, which may be the same or different, represented by R⁹;
n is 0, 1 or 2;
R⁹ is halogen, C₁-C₃alkyl, C₁-C₃haloalkyl, or C₁-C₃alkoxy;
R¹⁰ and R¹¹ are each independently selected from hydrogen and C₁-C₃alkyl;
R¹² is halogen, cyano, C₁-C₃alkyl, or C₁-C₃alkoxy;
or a salt or an N-oxide thereof.

2. The compound according to claim 1, wherein R¹ is heteroaryl, wherein the heteroaryl moiety is a 5- or 6-membered aromatic ring which comprises 1 or 2 heteroatoms individually selected from N and S, and wherein the heteroaryl moiety is optionally substituted with 1 or 2 groups, which may be the same or different, represented by R⁸.

3. The compound according to claim 1 or claim 2, wherein R¹ is heteroaryl, wherein the heteroaryl moiety is a 6-membered aromatic ring which comprises 1 or 2 nitrogen heteroatoms, and wherein the heteroaryl moiety is optionally substituted with 1 or 2 groups, which may be the same or different, represented by R⁸.

4. The compound according to any one of claims 1 to 3, wherein R² is halogen, cyano, acetyl, or N-methoxy-C-methyl-carbonimidoyl.

5. The compound according to any one of claims 1 to 4, wherein R³ is hydrogen or C₁-C₃alkyl.

6. The compound according to any one of claims 1 to 5, wherein R⁴, R⁵, and R⁶ are all hydrogen.

7. The compound according to any one of claims 1 to 6, wherein R⁸ is halogen, cyano, C₁-C₄alkyl, C₁-C₄alkoxy, C₁-C₃haloalkyl, C₁-C₃haloalkoxy, C₁-C₃alkylsulfanyl, or C₁-C₃alkylsulfonyl; or
any two adjacent R⁸ groups together with the carbon atoms to which they are attached, may form a 6-membered aryl ring, or any two adjacent R⁸ groups together with the carbon atoms to which they are attached, may form a 5- or 6-membered heterocyclyl ring, comprising 1 or 2 heteroatoms selected from O and N, or any two adjacent R⁸ groups together with the carbon atoms to which they are attached, may form a 5- or 6-membered heteroaryl ring, comprising 1 or 2 heteroatoms selected from O and N, and wherein the heterocyclyl or heteroaryl rings may be optionally substituted with 1, 2, or 3 groups, which may be the same or different, represented by R⁹.

8. The compound according to any one of claims 1 to 7 wherein R⁸ is halogen, C₁-C₃alkyl, C₁-C₃alkoxy, or C₁-C₃haloalkyl; or
any two adjacent R⁸ groups together with the carbon atoms to which they are attached, may form a 5- or 6-membered heterocyclyl ring, comprising 1 or 2 oxygen atoms, and wherein the heterocyclyl ring may be optionally substituted with 1 or 2 groups, which may be the same or different, represented by R⁹.

9. The compound according to any one of claims 1 to 7, wherein R⁹ is halogen.

10. The compound according to any one of claims 1 to 9, wherein X is O.

11. A herbicidal composition comprising a compound according to any one of the previous claims and an agriculturally acceptable formulation adjuvant.

12. A herbicidal composition according to claim 11, further comprising at least one additional pesticide.

13. A herbicidal composition according to claim 12, wherein the additional pesticide is a herbicide or herbicide safener.

14. A method of controlling unwanted plant growth, comprising applying a compound of Formula (I) as defined in any one of claims 1 to 10, or a herbicidal composition according to any one of claims 11 to 13, to the unwanted plants or to the locus thereof.

15. Use of a compound of Formula (I) according to any one of claims 1 to 10 as a herbicide.

## Patentansprüche

1. Verbindung der Formel (I): 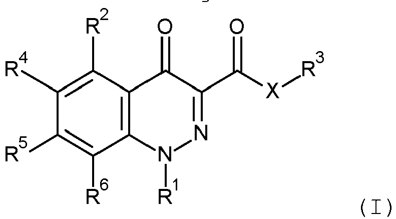 wobei
X für O, NR⁷ oder S steht;
R¹ für Heteroaryl steht, wobei es sich bei der Heteroarylgruppierung um einen 5- oder 6-gliedrigen aromatischen Ring, der 1, 2 oder 3 Heteroatome, die individuell aus N, O und S ausgewählt sind, umfasst, handelt und wobei die Heteroarylgruppierung gegebenenfalls durch 1, 2, 3 oder 4 durch R⁸ wiedergegebene Gruppen, die gleich oder verschieden sein können, substituiert ist;
R² für Halogen, Cyano, Cyano-C₁-C₆-alkyl, C₁-C₆-Halogenalkyl, C₁-C₆-Alkylcarbonyl, C₂-C₆-Alkenyl, C₂-C₆-Alkinyl, C₁-C₆-Alkoxy-C₂-C₆-alkenyl, C₂-C₆-Alkenyloxy-C₁-C₆-alkyl, -CR¹¹=N-OR¹⁰, Nitro, S(O)ₙ-C₁-C₆-Alkyl, S(O)ₙ-C₁-C₆-Halogenalkyl oder S(O)ₙ-C₃-C₆-Cycloalkyl steht;
R³ für Wasserstoff, C₁-C₆-Alkyl, C₁-C₆-Halogenalkyl, C₃-C₆-Cycloalkyl, C₃-C₆-Cycloalkyl-C₁-C₆-alkyl, C₁-C₆-Alkoxy-C₁-C₆-alkyl, C₂-C₆-Alkenyl, C₂-C₆-Alkinyl, Phenyl oder Phenyl-C₁-C₃-alkyl steht, wobei die Phenylgruppierungen gegebenenfalls durch 1, 2, 3 oder 4 durch R¹² wiedergegebene Gruppen, die gleich oder verschieden sein können, substituiert sein können;
R⁴, R⁵ und R⁶ jeweils unabhängig aus Wasserstoff, Halogen, Cyano, C₁-C₆-Alkyl, C₁-C₆-Alkoxy, C₁-C₆-Halogenalkyl, C₁-C₆-Halogenalkoxy, C₁-C₆-Alkylsulfanyl, C₁-C₆-Alkylsulfinyl und C₁-C₆-Alkylsulfonyl ausgewählt sind;
R⁷ für Wasserstoff, C₁-C₃-Alkyl oder C₁-C₃-Alkoxy steht;
R⁸ für Halogen, Cyano, C₁-C₆-Alkyl, C₁-C₆-Alkoxy, C₁-C₆-Halogenalkyl, C₁-C₆-Halogenalkoxy, C₁-C₆-Alkylsulfanyl oder C₁-C₆-Alkylsulfonyl steht; oder
beliebige zwei benachbarte R⁸-Gruppen zusammen mit den Kohlenstoffatomen, an die sie gebunden sind, einen 6-gliedrigen Arylring bilden können oder beliebige zwei benachbarte R⁸-Gruppen zusammen mit den Kohlenstoffatomen, an die sie gebunden sind, einen 5- oder 6-gliedrigen Heterocyclylring mit 1 oder 2 Heteroatomen, die aus O und N ausgewählt sind, bilden können oder beliebige zwei benachbarte R⁸-Gruppen zusammen mit den Kohlenstoffatomen, an die sie gebunden sind, einen 5- oder 6-gliedrigen Heteroarylring mit 1 oder 2 Heteroatomen, die aus O und N ausgewählt sind, bilden können und wobei die Aryl-, Heterocyclyl- oder Heteroarylringe gegebenenfalls durch 1, 2, 3 oder 4 durch R⁹ wiedergegebene Gruppen, die gleich oder verschieden sein können, substituiert sein können;
n für 0, 1 oder 2 steht;
R⁹ für Halogen, C₁-C₃-Alkyl, C₁-C₃-Halogenalkyl oder C₁-C₃-Alkoxy steht;
R¹⁰ und R¹¹ jeweils unabhängig aus Wasserstoff und C₁-C₃-Alkyl ausgewählt sind;
R¹² für Halogen, Cyano, C₁-C₃-Alkyl oder C₁-C₃-Alkoxy steht;
oder ein Salz oder ein N-Oxid davon.

2. Verbindung nach Anspruch 1, wobei R¹ für Heteroaryl steht, wobei es sich bei der Heteroarylgruppierung um einen 5- oder 6-gliedrigen aromatischen Ring, der 1 oder 2 Heteroatome, die individuell aus N und S ausgewählt sind, umfasst, handelt und wobei die Heteroarylgruppierung gegebenenfalls durch 1 oder 2 durch R⁸ wiedergegebene Gruppen, die gleich oder verschieden sein können, substituiert ist.

3. Verbindung nach Anspruch 1 oder Anspruch 2, wobei R¹ für Heteroaryl steht, wobei es sich bei der Heteroarylgruppierung um einen 6-gliedrigen aromatischen Ring, der 1 oder 2 Stickstoff-Heteroatome umfasst, handelt und wobei die Heteroarylgruppierung gegebenenfalls durch 1 oder 2 durch R⁸ wiedergegebene Gruppen, die gleich oder verschieden sein können, substituiert ist.

4. Verbindung nach einem der Ansprüche 1 bis 3, wobei R² für Halogen, Cyano, Acetyl oder N-Methoxy-C-methyl-carbonimidoyl steht.

5. Verbindung nach einem der Ansprüche 1 bis 4, wobei R³ für Wasserstoff oder C₁-C₃-Alkyl steht.

6. Verbindung nach einem der Ansprüche 1 bis 5, wobei R⁴, R⁵ und R⁶ alle für Wasserstoff stehen.

7. Verbindung nach einem der Ansprüche 1 bis 6, wobei R⁸ für Halogen, Cyano, C₁-C₄-Alkyl, C₁-C₄-Alkoxy, C₁-C₃-Halogenalkyl, C₁-C₃-Halogenalkoxy, C₁-C₃-Alkylsulfanyl oder C₁-C₃-Alkylsulfonyl steht; oder
beliebige zwei benachbarte R⁸-Gruppen zusammen mit den Kohlenstoffatomen, an die sie gebunden sind, einen 6-gliedrigen Arylring bilden können oder beliebige zwei benachbarte R⁸-Gruppen zusammen mit den Kohlenstoffatomen, an die sie gebunden sind, einen 5- oder 6-gliedrigen Heterocyclylring mit 1 oder 2 Heteroatomen, die aus O und N ausgewählt sind, bilden können oder beliebige zwei benachbarte R⁸-Gruppen zusammen mit den Kohlenstoffatomen, an die sie gebunden sind, einen 5- oder 6-gliedrigen Heteroarylring mit 1 oder 2 Heteroatomen, die aus O und N ausgewählt sind, bilden können und wobei die Heterocyclyl- oder Heteroarylringe gegebenenfalls durch 1, 2 oder 3 durch R⁹ wiedergegebene Gruppen, die gleich oder verschieden sein können, substituiert sein können.

8. Verbindung nach einem der Ansprüche 1 bis 7, wobei R⁸ für Halogen, C₁-C₃-Alkyl, C₁-C₃-Alkoxy oder C₁-C₃-Halogenalkyl steht; oder
beliebige zwei benachbarte R⁸-Gruppen zusammen mit den Kohlenstoffatomen, an die sie gebunden sind, einen 5- oder 6-gliedrigen Heterocyclylring mit 1 oder 2 Sauerstoffatomen bilden können und wobei der Heterocyclylring gegebenenfalls durch 1 oder 2 durch R⁹ wiedergegebene Gruppen, die gleich oder verschieden sein können, substituiert sein kann.

9. Verbindung nach einem der Ansprüche 1 bis 7, wobei R⁹ für Halogen steht.

10. Verbindung nach einem der Ansprüche 1 bis 9, wobei X für O steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von unerwünschtem Pflanzenwuchs, bei dem man eine wie in einem der Ansprüche 1 bis 10 definierte Verbindung der Formel (I) oder eine herbizide Zusammensetzung nach einem der Ansprüche 11 bis 13 auf die unerwünschten Pflanzen oder deren Standort ausbringt.

15. Verwendung einer Verbindung der Formel (I) nach einem der Ansprüche 1 bis 10 als Herbizid.

## Revendications

1. Composé de formule (I) : 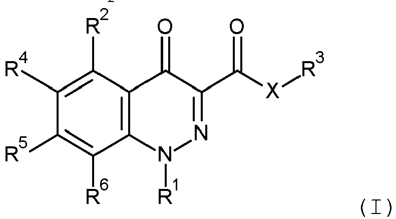
X étant O, NR⁷ ou S ;
R¹ étant hétéroaryle, le fragment hétéroaryle étant un cycle aromatique à 5 ou 6 chaînons qui comprend 1, 2 ou 3 hétéroatomes individuellement choisis parmi N, O et S et le fragment hétéroaryle étant éventuellement substitué par 1, 2, 3 ou 4 groupes, qui peuvent être identiques ou différents, représentés par R⁸ ;
R² étant halogène, cyano, cyano-alkyle en C₁-C₆, halogénoalkyle en C₁-C₆, alkyl en C₁-C₆-carbonyle, alcényle en C₂-C₆, alcynyle en C₂-C₆, alcoxy en C₁-C₆-alcényle en C₂-C₆, alcényloxy en C₂-C₆-alkyle en C₁-C₆, - CR¹¹=N-OR¹⁰, nitro, S(O)ₙ-alkyle en C₁-C₆, S(O)ₙ-halogénoalkyle en C₁-C₆ ou S(O)ₙ-cycloalkyle en C₃-C₆ ;
R³ étant hydrogène, alkyle en C₁-C₆, halogénoalkyle en C₁-C₆, cycloalkyle en C₃-C₆, cycloalkyl en C₃-C₆-alkyle en C₁-C₆, alcoxy en C₁-C₆-alkyle en C₁-C₆, alcényle en C₂-C₆, alcynyle en C₂-C₆, phényle ou phényl-alkyle en C₁-C₃, les fragments phényle pouvant être éventuellement substitués par 1, 2, 3 ou 4 groupes, qui peuvent être identiques ou différents, représentés par R¹² ;
R⁴, R⁵ et R⁶ étant chacun indépendamment choisis parmi hydrogène, halogène, cyano, alkyle en C₁-C₆, alcoxy en C₁-C₆, halogénoalkyle en C₁-C₆, halogénoalcoxy en C₁-C₆, alkylsulfanyle en C₁-C₆, alkylsulfinyle en C₁-C₆ et alkylsulfonyle en C₁-C₆ ;
R⁷ étant hydrogène, alkyle en C₁-C₃ ou alcoxy en C₁-C₃ ;
R⁸ étant halogène, cyano, alkyle en C₁-C₆, alcoxy en C₁-C₆, halogénoalkyle en C₁-C₆, halogénoalcoxy en C₁-C₆, alkylsulfanyle en C₁-C₆ ou alkylsulfonyle en C₁-C₆ ; ou
deux groupes R⁸ adjacents quelconques, conjointement avec les atomes de carbone auxquels ils sont attachés, pouvant former un cycle aryle à 6 chaînons ou deux groupes R⁸ adjacents quelconques, conjointement avec les atomes de carbone auxquels ils sont attachés, pouvant former un cycle hétérocyclyle à 5 ou 6 chaînons, comprenant 1 ou 2 hétéroatomes choisis entre O et N, ou deux groupes R⁸ adjacents quelconques, conjointement avec les atomes de carbone auxquels ils sont attachés, pouvant former un cycle hétéroaryle à 5 ou 6 chaînons, comprenant 1 ou 2 hétéroatomes choisis entre O et N, les cycles aryle, hétérocyclyle ou hétéroaryle pouvant être éventuellement substitués par 1, 2, 3 ou 4 groupes, qui peuvent être identiques ou différents, représentés par R⁹ ;
n étant 0, 1 ou 2 ;
R⁹ étant halogène, alkyle en C₁-C₃, halogénoalkyle en C₁-C₃ ou alcoxy en C₁-C₃ ;
R¹⁰ et R¹¹ étant chacun indépendamment choisis entre hydrogène et alkyle en C₁-C₃ ;
R¹² étant halogène, cyano, alkyle en C₁-C₃ ou alcoxy en C₁-C₃ ;
ou sel ou N-oxyde de celui-ci.

2. Composé selon la revendication 1, R¹ étant hétéroaryle, le fragment hétéroaryle étant un cycle aromatique à 5 ou 6 chaînons qui comprend 1 ou 2 hétéroatomes individuellement choisis entre N et S et le fragment hétéroaryle étant éventuellement substitué par 1 ou 2 groupes, qui peuvent être identiques ou différents, représentés par R⁸.

3. Composé selon la revendication 1 ou la revendication 2, R¹ étant hétéroaryle, le fragment hétéroaryle étant un cycle aromatique à 6 chaînons qui comprend 1 ou 2 hétéroatomes azote et le fragment hétéroaryle étant éventuellement substitué par 1 ou 2 groupes, qui peuvent être identiques ou différents, représentés par R⁸.

4. Composé selon l'une quelconque des revendications 1 à 3, R² étant halogène, cyano, acétyle ou N-méthoxy-C-méthyl-carbonimidoyle.

5. Composé selon l'une quelconque des revendications 1 à 4, R³ étant hydrogène ou alkyle en C₁-C₃.

6. Composé selon l'une quelconque des revendications 1 à 5, R⁴, R⁵ et R⁶ étant tous hydrogène.

7. Composé selon l'une quelconque des revendications 1 à 6, R⁸ étant halogène, cyano, alkyle en C₁-C₄, alcoxy en C₁-C₄, halogénoalkyle en C₁-C₃, halogénoalcoxy en C₁-C₃ alkylsulfanyle en C₁-C₃ ou alkylsulfonyle en C₁-C₃ ; ou deux groupes R⁸ adjacents quelconques, conjointement avec les atomes de carbone auxquels ils sont attachés, pouvant former un cycle aryle à 6 chaînons ou deux groupes R⁸ adjacents quelconques, conjointement avec les atomes de carbone auxquels ils sont attachés, pouvant former un cycle hétérocyclyle à 5 ou 6 chaînons, comprenant 1 ou 2 hétéroatomes choisis entre O et N, ou deux groupes R⁸ adjacents quelconques, conjointement avec les atomes de carbone auxquels ils sont attachés, pouvant former un cycle hétéroaryle à 5 ou 6 chaînons, comprenant 1 ou 2 hétéroatomes choisis entre O et N, les cycles hétérocyclyle ou hétéroaryle pouvant être éventuellement substitués par 1, 2 ou 3 groupes, qui peuvent être identiques ou différents, représentés par R⁹.

8. Composé selon l'une quelconque des revendications 1 à 7, R⁸ étant halogène, alkyle en C₁-C₃, alcoxy en C₁-C₃ ou halogénoalkyle en C₁-C₃ ; ou
deux groupes R⁸ adjacents quelconques, conjointement avec les atomes de carbone auxquels ils sont attachés, pouvant former un cycle hétérocyclyle à 5 ou 6 chaînons, comprenant 1 ou 2 atomes d'oxygène, le cycle hétérocyclyle pouvant être éventuellement substitué par 1 ou 2 groupes, qui peuvent être identiques ou différents, représentés par R⁹.

9. Composé selon l'une quelconque des revendications 1 à 7, R⁹ étant halogène.

10. Composé selon l'une quelconque des revendications 1 à 9, X étant O.

11. Composition herbicide comprenant un composé selon l'une quelconque des revendications précédentes et un adjuvant de formulation acceptable sur le plan agricole.

12. Composition herbicide selon la revendication 11, comprenant en outre au moins un pesticide supplémentaire.

13. Composition herbicide selon la revendication 12, le pesticide supplémentaire étant un herbicide ou un phytoprotecteur herbicide.

14. Procédé de lutte contre une croissance végétale non souhaitée, comprenant l'application d'un composé de formule (I) tel que défini dans l'une quelconque des revendications 1 à 10, ou d'une composition herbicide selon l'une quelconque des revendications 11 à 13, sur les végétaux non souhaités ou sur le lieu où ils se développent.

15. Utilisation d'un composé de formule (I) selon l'une quelconque des revendications 1 à 10 en tant qu'herbicide.
